(19) Europäisches Patentamt
European Patent Office
Office européen des brevets

(11) Veröffentlichungsnummer: **0 332 064**
**A2**

# (12) EUROPÄISCHE PATENTANMELDUNG

(21) Anmeldenummer: 89103721.0

(22) Anmeldetag: 03.03.89

(51) Int. Cl.4: C07C 93/14 , C07C 143/68 , C07C 103/30 , C07C 121/78 , C07D 317/64 , A61K 31/135 , A61K 31/16 , A61K 31/335

(30) Priorität: 10.03.88 DE 3807813

(43) Veröffentlichungstag der Anmeldung: 13.09.89 Patentblatt 89/37

(84) Benannte Vertragsstaaten: AT BE CH DE ES FR GB GR IT LI LU NL SE

(71) Anmelder: **Dr. Karl Thomae GmbH**
**Postfach 1755**
**D-7950 Biberach 1(DE)**

(72) Erfinder: **Psiorz, Manfred, Dr. Dipl.-Chem.**
**Bergstrasse 5**
**D-7957 Schemmerhofen-Alberweiler(DE)**
Erfinder: **Reiffen, Manfred, Dr. Dipl.-Chem.**
**Matthias-Erzberger-Strasse 40**
**D-7950 Biberach 1(DE)**
Erfinder: **Heider, Joachim, Dr. Dipl.-Chem.**
**Am Hang 3**
**D-7951 Warthausen 1(DE)**
Erfinder: **Bomhard, Andreas, Dr. Dipl.-Chem.**
**Dinglingerstrasse 9**
**D-7950 Biberach 1(DE)**
Erfinder: **Dämmgen, Jürgen, Dr.**
**Eichweg 7**
**D-7951 Sulmingen(DE)**
Erfinder: **Lillie, Christian, Dr.**
**Hansi-Niese-Weg 12**
**A-1130 Wien(AT)**
Erfinder: **Kobinger, Walter, Dr.**
**Belghofergasse 27**
**A-1121 Wien(AT)**
Erfinder: **Trach, Volker, Dr.**
**Probststrasse 7**
**D-7950 Biberach 1(DE)**

(54) **Neue Benzocycloheptenderivate, diese Verbindungen enthaltende Arzneimittel und Verfahren zu deren Herstellung.**

(57) Die vorliegende Erfindung betrifft Benzocycloheptenderivate der Formel

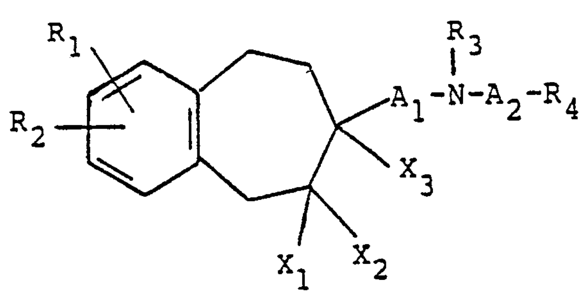

,(I)

in der
$X_1$ ein Wasserstoffatom, $X_2$ ein Wasserstoffatom und $X_3$ ein Wasserstoffatom, eine Hydroxy- oder Alkoxygruppe oder
$X_1$ und $X_3$ zusammen eine weitere Kohlenstoff-Kohlenstoff-Bindung und $X_2$ ein Wasserstoffatom oder
$X_1$ und $X_2$ zusammen mit dem dazwischenliegenden Kohlenstoffatom eine Carbonylgruppe und $X_3$ ein

EP 0 332 064 A2

Wasserstoffatom,
$A_1$ eine gegebenenfalls durch eine Alkylgruppe substituierte geradkettige Alkylengruppe, in welcher eine im Rest $A_1$ enthaltene mit dem Benzocycloheptenring verknüpfte Ethylengruppe durch eine Ethenylen- oder Ethinylengruppe ersetzt sein kann,
$A_2$ eine gegebenenfalls durch eine Alkylgruppe substituierte geradkettige Alkylengruppe, in welcher, falls n die Zahl 0 darstellt, eine in dem Rest $A_2$ enthaltene Ethylen gruppe, welche mit dem Rest $R_4$ verknüpft ist, durch eine Ethenylengruppe ersetzt sein kann,
$R_1$ ein Wasserstoff- oder Halogenatom, eine Trifluormethyl-, Nitro-, Amino-, Alkylamino-, Dialkylamino-, Alkyl-, Hydroxy-, Alkoxy- oder Phenylalkoxygruppe,
$R_2$ ein Wasserstoffatom oder Halogenatom, eine Hydroxy-, Alkoxy-, Phenylalkoxy- oder Alkylgruppe, oder
$R_1$ und $R_2$ zusammen eine Alkylendioxygruppe,
$R_3$ ein Wasserstoffatom, eine Alkylgruppe oder eine Alkenylgruppe und
$R_4$ eine Gruppe der Formel

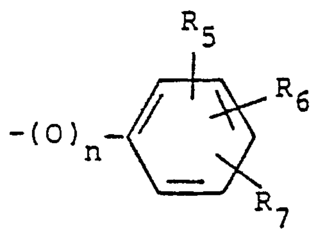

, wobei

n die Zahl 0 oder 1,
$R_5$ ein Wasserstoff- oder Halogenatom, eine Alkyl-, Nitro-, Amino-, Alkylamino-, Dialkylamino-, Alkanoylamino-, Alkoxycarbonylamino-, Alkylsulfonylamino-, Bis(alkylsulfonyl) amino-, N-Alkyl-alkylsulfonylamino-, Cyano-, Alkylmercapto-, Alkylsulfinyl- oder Alkylsulfonylgruppe oder eine gegebenenfalls durch eine Alkyl-, Phenylalkyl, 2-Hydroxyäthyl-, 3-Hydroxy-n-propyl, 2-Hydroxy-n-propyl, Alkylsulfonyl-, Cyanoalkyl-, Alkoxycarbonyl, Hydroxycarbonylalkyl, Alkoxycarbonylalkyl-, Trifluormethyl, Difluormethyl- oder Trifluormethylsulfonylgruppe substituierte Hydroxygruppe,
$R_6$ ein Wasserstoff- oder Halogenatom, eine Alkyl-, Hydroxy-, Alkoxy-, Cyano- oder Trifluormethylgruppe oder
$R_5$ und $R_6$ zusammen eine Alkylendioxygruppe und
$R_7$ ein Wasserstoff- oder Halogenatom, eine Alkyl- oder Alkoxygruppe bedeuten, deren Enantiomeren, deren Diastereomeren und deren Säureadditionssalze mit anorganischen oder organischen Säuren, welche wertvolle pharmakologische Eigenschaften aufweisen, insbesondere herzfrequenzsenkende, blutdrucksenkende, $Ca^{++}$-antagonistische und/oder antithrombotische Wirkung sowie eine antiischämische Wirkung am Herzen und eine den $O_2$-Bedarf des Herzens vermindernde Wirkung.
Die neuen Verbindungen können nach an sich bekannten Verfahren hergestellt werden.

## Neue Benzocycloheptenderivate, diese Verbindungen enthaltende Arzneimittel und Verfahren zu deren Herstellung

In der EP-A-0.177.960 werden bereits Tetrahydronaphthaline, welche in 2-Stellung durch eine gegebenenfalls durch einen Acylrest substituierte Hydroxygruppe substituiert sind, beschrieben. Diese Verbindungen besitzen eine ausgeprägte Calcium-antagonistische Wirkung und können deshalb als Arzneimittel verwendet werden, insbesondere für die Bekämpfung bzw. Verhütung von Angina pectoris, Ischämie, Arrhythmien und Bluthochdruck.

Überraschenderweise wurde nun gefunden, daß die neuen Benzocycloheptenderivate der Formel

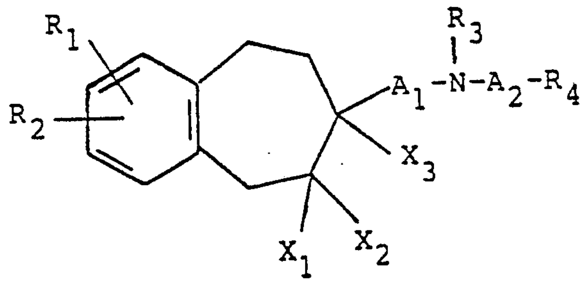

,(I)

deren Enantiomeren, deren Diastereomeren und deren Säureadditionssalze, insbesondere deren physiologisch verträgliche Säureadditionssalze mit anorganischen oder organischen Säuren, andere wertvolle pharmakologische Eigenschaften aufweisen, insbesondere eine herzfreguenzsenkende Wirkung und eine Verminderung des $0_2$-Bedarfes des Herzens.

Gegenstand der vorliegenden Erfindung sind somit die neuen Benzocycloheptenderivate der obigen Formel I, deren Enantiomeren, deren Diastereomeren, deren Säureadditionssalze, insbesondere für die pharmazeutische Verwendung deren physiologisch verträgliche Säureadditionssalze mit anorganischen oder organischen Säuren, Verfahren zu ihrer Herstellung und diese Verbindungen enthaltende Arzneimittel.

Die ungesättigten Verbindungen der Formel I stellen auch Zwischenprodukte zur Herstellung der gesättigten Verbindungen der Formel I dar.

In der obigen allgemeinen Formel I bedeuten

$X_1$ ein Wasserstoffatom, $X_2$ ein Wasserstoffatom und $X_3$ ein Wasserstoffatom, eine Hydroxy- oder Alkoxygruppe mit 1 bis 3 Kohlenstoffatomen oder

$X_1$ und $X_3$ zusammen eine weitere Kohlenstoff-Kohlenstoff-Bindung und $X_2$ ein Wasserstoffatom oder

$X_1$ und $X_2$ zusammen mit dem dazwischenliegenden Kohlenstoffatom eine Carbonylgruppe und $X_3$ ein Wasserstoffatom,

$A_1$ eine gegebenenfalls durch eine Alkylgruppe mit 1 bis 3 Kohlenstoffatomen substituierte geradkettige Alkylengruppe mit 3 oder 4 Kohlenstoffatomen, in welcher eine im Rest $A_1$ enthaltene mit dem Benzocycloheptenring verknüpfte Ethylengruppe durch eine Ethenylen- oder Ethinylengruppe ersetzt sein kann,

$A_2$ eine gegebenenfalls durch eine Alkylgruppe mit 1 bis 3 Kohlenstoffatomen substituierte geradkettige Alkylengruppe mit 2 bis 5 Kohlenstoffatomen, in welcher, falls n die Zahl 0 darstellt, eine in dem Rest $A_2$ enthaltene Ethylengruppe, welche mit dem Rest $R_4$ verknüpft ist, durch eine Ethenylengruppe ersetzt sein kann,

$R_1$ ein Wasserstoff- oder Halogenatom, eine Trifluormethyl-, Nitro-, Amino-, Alkylamino-, Dialkylamino-, Alkyl-, Hydroxy-, Alkoxy- oder Phenylalkoxygruppe,

$R_2$ ein Wasserstoffatom oder Halogenatom, eine Hydroxy-, Alkoxy-, Phenylalkoxy- oder Alkylgruppe, oder

$R_1$ und $R_2$ zusammen eine Alkylendioxygruppe mit 1 oder 2 Kohlenstoffatomen,

$R_3$ ein Wasserstoffatom, eine Alkylgruppe oder eine Alkenylgruppe mit 3 bis 5 Kohlenstoffatomen und

$R_4$ eine Gruppe der Formel

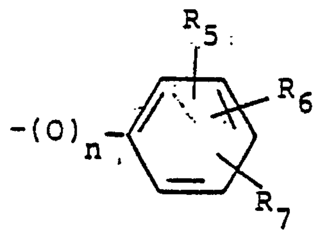

, wobei

n die Zahl 0 oder 1,
$R_5$ ein Wasserstoff- oder Halogenatom, eine Alkyl-, Nitro-, Amino-, Alkylamino-, Dialkylamino-, Alkanoylamino-, Alkoxycarbonylamino-, Alkylsulfonylamino-, Bis(alkylsulfonyl)-amino-, N-Alkyl-alkylsulfonylamino-, Cyano-, Alkylmercapto-, Alkylsulfinyl- oder Alkylsulfonylgruppe oder eine gegebenenfalls durch eine Alkyl-, Phenylalkyl-, 2-Hydroxyäthyl-, 3-Hydroxy-n-propyl-, 2-Hydroxy-n-propyl-, Alkylsulfonyl-, Cyanoalkyl-, Alkoxycarbonyl-, Hydroxycarbonylalkyl-, Alkoxycar bonylalkyl, Trifluormethyl-, Difluormethyl- oder Trifluormethylsulfonylgruppe substituierte Hydroxygruppe,
$R_6$ ein Wasserstoff- oder Halogenatom, eine Alkyl-, Hydroxy-, Alkoxy-, Cyano- oder Trifluormethylgruppe oder
$R_5$ und $R_6$ zusammen eine Alkylendioxygruppe mit 1 oder 2 Kohlenstoffatomen und
$R_7$ ein Wasserstoff- oder Halogenatom, eine Alkyl- oder Alkoxygruppe bedeuten, wobei alle vorstehend erwähnten Alkyl- oder Alkoxygruppen jeweils 1 bis 3 Kohlenstoffatome und die vorstehend erwähnten Alkanoylgruppen jeweils 2 oder 3 Kohlenstoffatome enthalten können.

Fur die bei der Definition der Reste eingangs erwähnten Bedeutungen kommt beispielsweise
für $R_1$ die des Wasserstoff-, Fluor-, Chlor- oder Bromatoms, der Methyl-, Ethyl-, n-Propyl-, Isopropyl-, Trifluormethyl-, Hydroxy-, Methoxy-, Ethoxy-, n-Propoxy-, Isopropoxy-, Nitro-, Amino-, Methylamino-, Ethylamino-, n-Propylamino-, Isopropylamino-, Dimethylamino-, Diethylamino-, Di-n-propylamino-, Diisopropylamino-, Methyl-ethylamino-, Methyl-n-propylamino-, Methyl-isopropylamino-, Ethyl-n-propylamino-, Benzyloxy-, 1-Phenylethoxy-, 1-Phenylpropoxy-, 2-Phenylethoxy- oder 3-Phenylpropoxygruppe,
für $R_2$ die des Wasserstoff-, Chlor- oder Bromatoms, der Methyl-, Ethyl-, n-Propyl-, Isopropyl-, Hydroxy-, Methoxy-, Ethoxy-, n-Propoxy-, Isopropoxy-, Benzyloxy-, 1-Phenylethoxy-, 2-Phenylethoxy-, 2-Phenylpropoxy- oder 3-Phenylpropoxygruppe oder zusammen mit $R_1$ die der Methylendioxy- oder Ethylendioxygruppe,
für $R_3$ die des Wasserstoffatoms, der Methyl-, Ethyl-, n-Propyl-, Isopropyl-, Allyl-, Crotyl- oder n-Penten-2-yl-gruppe,
für $R_5$ die des Wasserstoff-, Fluor-, Chlor-, Brom- oder Jodatoms, der Methyl-, Ethyl-, n-Propyl-, Isopropyl-, Hydroxy-, Methoxy-, Ethoxy-, n-Propoxy-, Isopropoxy-, Amino-, Methylamino-, Ethylamino-, n-Propylamino-, Isopropylamino-, Dimethylamino-, Diethylamino-, Di-n-propylamino-, Diisopropylamino-, Methyl-ethylamino-, Methyl-n-propylamino-, Methyl-isopropylamino-, Ethyl-n-propylamino-, Acetylamino-, Propionylamino-, Methoxycarbonylamino-, Ethoxycarbonylamino-, n-Propoxycarbonylamino-, Methylsulfonylamino-, Ethylsulfonylamino-, n-Propylsulfonylamino-, Bis(methylsulfonyl)-amino-, Bis(ethylsulfonyl)-amino-, N-Methyl-methylsulfonylamino-, Cyano-, Methylmercapto-, Ethylmercapto-, n-Propylmercapto-, Methylsulfinyl-, Ethylsulfinyl-, Isopropylsulfinyl-, Methylsulfonyl-, Ethylsulfonyl-, n-Propylsulfonyl-, Benzyloxy-, 1-Phenylethoxy-, 2-Phenylethoxy-, 2-Phenyl-propoxy-, 3-Phenylpropoxy-, 2-Hydroxy-ethoxy-, 2-Hydroxy-n-propoxy-, 3-Hydroxy-n-propoxy-, Methylsulfonyloxy-, Ethylsulfonyloxy-, Isopropylsulfonyloxy-, Methoxycarbonyloxy-, Ethoxycarbonyloxy-, Isopropoxycarbonyloxy-, Hydroxycarbonylmethoxy-, 2-(Hydroxycarbonyl)-ethoxy-, Methoxycarbonylmethoxy-, Ethoxycarbonylmethoxy-, Isopropoxycarbonylmethoxy-, 2-(Methoxycarbonyl)-ethoxy-, 2-(n-Propoxycarbonyl)-ethoxy-, Cyanomethoxy-, 2-Cyanoethoxy-, 3-Cyano-n-propoxy-, Difluormethoxy-, Trifluormethoxy- oder Nitrogruppe,
für $R_6$ die des Wasserstoff-, Chlor- oder Bromatoms, der Methyl-, Ethyl-, n-Propyl-, Isopropyl-, Hydroxy-, Methoxy-, Ethoxy-, n-Propoxy-, Isopropoxy-, Cyano- oder Trifluormethylgruppe oder $R_5$ und $R_6$ zusammen die der Methylendioxy-oder Ethylendioxygruppe,
für $R_7$ die des Wasserstoffatoms, der Hydroxy-, Methoxy-, Ethoxy-, n-Propoxy- oder Isopropoxygruppe,
für $A_1$ die der n-Propylen-, 1-Methyl-n-propylen, 2-Methyl-n-propylen-, 3-Methyl-n-propylen-, 1-Ethyl-n-propylen-, 2-n-Propyl-n-propylen-, 3-Ethyl-n-propylen-, n-Butylen-, 1-Methyl-n-butylen-, 1-Ethyl-n-butylen-, Prop-1-enylen-, n-But-1-enylen-, 1-Methyl-prop-1-enylen-, 2-Methyl-prop-1-enylen-, 3-Methyl-prop-1-enylen-, Prop-1-inylen-, 3-Methyl-prop-1-inylen- oder n-But-1-inylengruppe und
für $A_2$ die der Ethylen-, 1-Methyl-ethylen-, 1-Ethyl-ethylen-, 1-Propyl-ethylen-, 2-Methyl-ethylen-, 2-Ethyl-ethylen-, n-Propylen-, n-Butylen-, n-Pentylen-, 1-Methyl-n-propylen-, 1-Methyl-n-butylen-, 1-Ethyl-n-propylen-, 2-Ethyl-n-propylen-, 1-Ethyl-n-butylen-, Prop-1-enylen-, n-But-1-enylen-, n-Pent-1-enylen-, 1-Methyl-prop-1-enylen, 2-Methyl-prop-1-enylen-, 3-Methyl-prop-1-enylen-, 4-Methyl-n-but-1-enylen- oder 5-Methyl-n-pent-1-enylengruppe in Betracht.

Beispielsweise seien zusätzlich zu den in den Beispielen genannten Verbindungen noch folgende Verbindungen genannt, die unter die vorstehend erwähnte Formel I fallen:

2,3-Dimethoxy-7-hydroxy-7-[3-(N-(2-(3, 4-dimethoxy-phenyl)-ethyl)-amino)-propin-1-yl]-6,7,8,9-tetrahydro-5H-benzocyclohepten,

2,3-Dimethoxy-7-hydroxy-7-[(3-(N-cinnamyl-N-methyl)-amino)-propin-1-yl]-6,7,8,9-tetrahydro-5H-benzocyclohepten,

2,3-Dimethoxy-7-hydroxy-7-[3-(N-(2-(4-benzyloxy-phenyl)-ethyl)-amino)-propin-1-yl]-6,7,8,9-tetrahydro-5H-benzocyclohepten,

2,3-Dimethoxy-7-hydroxy-7-[3-(N-(2-(4-methoxy-phenyl)-ethyl)-amino)-propin-1-yl]-6,7,8,9-tetrahydro-5H-benzocyclohepten,

2,3-Dimethoxy-7-hydroxy-7-[3-(N-(2-(3-methoxy-phenyl)-ethyl)-amino)-propin-1-yl]-6,7,8,9-tetrahydro-5H-benzocyclohepten,

2,3-Dimethoxy-7-hydroxy-7-[3-(N-(3-(3,4-dimethyl-phenoxy)-propyl)-amino)-propin-1-yl]-6,7,8,9-tetrahydro-5H-benzocyclohepten,

2,3-Dimethoxy-7-hydroxy-7-[3-(N-(3-(3-methoxy-phenoxy)-propyl)-amino)-propin-1-yl]-6,7,8,9-tetrahydro-5H-benzocyclohepten,

2,3-Dimethoxy-7-hydroxy-7-[3-(N-(2-(3-benzyloxy-phenyl)-ethyl)-amino)-propin-1-yl]-6,7,8,9-tetrahydro-5H-benzocyclohepten,

2,3-Dimethoxy-7-hydroxy-7-[3-(N-(2-(3-benzyloxy-phenyl)-ethyl)-amino)-propen-1-yl]-6,7,8,9-tetrahydro-5H-benzocyclohepten,

2,3-Dimethoxy-7-hydroxy-7-[3-(N-(2-phenylethyl)-amino)-propin-1-yl]-6,7,8,9-tetrahydro-5H-benzocyclohepten,

2,3-Dimethoxy-7-hydroxy-7-[3-(N-(3-phenylpropyl)-amino)-propin-1-yl]-6,7,8,9-tetrahydro-5H-benzocyclohepten,

2,3-Dimethoxy-7-hydroxy-7-[3-(N-(2-(3-methyl-phenyl)-ethyl)-amino)-propin-1-yl]-6,7,8,9-tetrahydro-5H-benzocyclohepten,

2,3-Dimethoxy-7-methoxy-7-[3-(N-methyl-N- (2-(3,4-dimethoxy-phenyl)-ethyl)-amino)-propin-1-yl] -6,7,8,9-tetrahydro-5H-benzocyclohepten,

2,3-Dimethoxy-7-hydroxy-7-[3-(N-methyl-N-(2-(3,4-dimethoxy-phenyl)-ethyl)-amino)-propin-1-yl]-6,7,8,9-tetrahydro-5H-benzocyclohepten,

2,3-Dimethoxy-7-hydroxy-7-[(3-(N-cinnamyl-N-methyl)-amino)-propin-1-yl] -6,7,8,9-tetrahydro-5H-benzocyclohepten,

2,3-Dimethoxy-7-hydroxy-7-[3-(N-methyl-N-(2-(3,5-dimethoxy-phenyl)-ethyl)-amino)-propin-1-yl]-6,7,8,9-tetrahydro-5H-benzocyclohepten,

2,3-Dimethoxy-7-hydroxy-7-[3-(N-methyl-N-(2-(4-benzyloxy-phenyl)-ethyl)-amino)-propin-1-yl]-6,7,8,9-tetrahydro-5H-benzocyclohepten,

2,3-Dimethoxy-7-hydroxy-7-[3-(N-methyl-N-(2-(4-methoxy-phenyl)-ethyl)-amino)-propin-1-yl]-6,7,8,9-tetrahydro-5H-benzocyclohepten,

2,3-Dimethoxy-7-hydroxy-7-[3-(N-methyl-N-(2-(3-methoxy-phenyl)-ethyl)-amino)-propin-1-yl]-6,7,8,9-tetrahydro-5H-benzocyclohepten,

2,3-Dimethoxy-7-hydroxy-7-[3-(N-methyl-N-(3-(3,4-dimethyl-phenoxy)-propyl)-amino)-propin-1-yl]-6,7,8,9-tetrahydro-5H-benzocyclohepten,

2,3-Dimethoxy-7-hydroxy-7-[3-(N-methyl-N-(3-(3-methoxy-phenoxy)-propyl)-amino)-propin-1-yl]-6,7,8,9-tetrahydro-5H-benzocyclohepten,

2,3-Dimethoxy-7-hydroxy-7-[3-(N-methyl-N-(2-(3-benzyloxy-phenyl)-ethyl)-amino)-propin-1-yl]-6,7,8,9-tetrahydro-5H-benzocyclohepten,

2,3-Dimethoxy-7-hydroxy-7-[3-(N-methyl-N-(2-(3-benzyloxy-phenyl)-ethyl)-amino)-propen-1-yl]-6,7,8,9-tetrahydro-5H-benzocyclohepten,

2,3-Dimethoxy-7-hydroxy-7-[3-(N-methyl-N-(2-phenylethyl)-amino)-propin-1-yl]-6,7,8,9-tetrahydro-5H-benzocyclohepten,

2,3-Dimethoxy-7-hydroxy-7-[3-(N-methyl-N-(3-phenylpropyl)-amino)-propin-1-yl]-6,7,8,9-tetrahydro-5H-benzocyclohepten,

2,3-Dimethoxy-7-hydroxy-7-[3-(N-methyl-N-(2-(3-methyl-phenyl)-ethyl)-amino)-propin-1-yl]-6,7,8,9-tetrahydro-5H-benzocyclohepten,

2,3-Dimethoxy-7-methoxy-7-[3-(N-ethyl-N-(2-(3,4-dimethoxy-phenyl)-ethyl)-amino)-propin-1-yl]-6,7,8,9-tetrahydro-5H-benzocyclohepten,
2,3-Dimethoxy-7-hydroxy-7-[3-(N-ethyl-N-(2-(3,4-dimethoxy-phenyl)-ethyl)-amino)-propin-1-yl]-6,7,8,9-tetrahydro-5H-benzocyclohepten,
7-Methoxy-7-[3-(N-(2-(3,4-dimethoxy-phenyl)-ethyl)-amino)-propin-1-yl]-6,7,8,9-tetrahydro-5H-benzocyclohepten,
7-Hydroxy-7-[3-(N-(2-(3,4-dimethoxy-phenyl)-ethyl)-amino)-propin-1-yl]-6,7,8,9-tetrahydro-5H-benzocyclohepten,
7-Hydroxy-7-[3-(N-(2-(4-benzyloxy-phenyl)-ethyl)-amino)-propin-1-yl]-6,7,8,9-tetrahydro-5H-benzocyclohepten,
7-Hydroxy-7-[3-(N-(2-(4-methoxy-phenyl)-ethyl)-amino)-propin-1-yl]-6,7,8,9-tetrahydro-5H-benzocyclohepten,
7-Hydroxy-7-[3-(N-(2-(3-methoxy-phenyl)-ethyl)-amino)-propin-1-yl]-6,7,8,9-tetrahydro-5H-benzocyclohepten,
7-Hydroxy-7-[3-(N-(3-(3,4-dimethyl-phenoxy)-propyl)-amino)-propin-1-yl]-6,7,8,9-tetrahydro-5H-benzocyclohepten,
7-Hydroxy-7-[3-(N-(3-(3-methoxy-phenoxy)-propyl)-amino)-propin-1-yl]-6,7,8,9-tetrahydro-5H-benzocyclohepten,
7-Hydroxy-7-[3-(N-(2-(3-benzyloxy-phenyl)-ethyl)-amino)-propin-1-yl]-6,7,8,9-tetrahydro-5H-benzocyclohepten,
7-Hydroxy-7-[3-(N-(2-(3-benzyloxy-phenyl)-ethyl)-amino)-propen-1-yl]-6,7,8,9-tetrahydro-5H-benzocyclohepten,
7-Hydroxy-7-[3-(N-(2-phenylethyl)-amino)-propin-1-yl]-6,7,8,9-tetrahydro-5H-benzocyclohepten,
7-Hydroxy-7-[3-(N-(3-phenylpropyl)-amino)-propin-1-yl]-6,7,8,9-tetrahydro-5H-benzocyclohepten,
7-Hydroxy-7-[3-(N-(2-(3-methyl-phenyl)-ethyl)-amino)-propin-1-yl]-6,7,8,9-tetrahydro-5H-benzocyclohepten,
7-Methoxy-7-[3-(N-methyl-N-(2-(3,4-dimethoxy-phenyl)-ethyl)-amino)-propin-1-yl]-6,7,8,9-tetrahydro-5H-benzocyclohepten,
7-Hydroxy-7-[3-(N-methyl-N-(2-(3,4-dimethoxy-phenyl)-ethyl)-amino)-propin-1-yl]-6,7,8,9-tetrahydro-5H-benzocyclohepten,
7-Hydroxy-7-[(3-(N-cinnamyl-N-methyl)-amino)-propin-1-yl]-6,7,8,9-tetrahydro-5H-benzocyclohepten,
7-Hydroxy-7-[3-(N-methyl-N-(2-(3,5-dimethoxy-phenyl)-ethyl)-amino)-propin-1-yl]-6,7,8,9-tetrahydro-5H-benzocyclohepten,
7-Hydroxy-7-[3-(N-methyl-N-(2-(4-benzyloxy-phenyl)-ethyl)-amino)-propin-1-yl]-6,7,8,9-tetrahydro-5H-benzocyclohepten,
7-Hydroxy-7-[3-(N-methyl-N-(2-(4-methoxy-phenyl)-ethyl)-amino)-propin-1-yl]-6,7,8,9-tetrahydro-5H-benzocyclohepten,
7-Hydroxy-7-[3-(N-methyl-N-(2-(3-methoxy-phenyl)-ethyl)-amino)-propin-1-yl]-6,7,8,9-tetrahydro-5H-benzocyclohepten,
7-Hydroxy-7-[3-(N-methyl-N-(3-(3,4-dimethyl-phenoxy)-propyl)-amino)-propin-1-yl]-6,7,8,9-tetrahydro-5H-benzocyclohepten,
7-Hydroxy-7-[3-(N-methyl-N-(3-(3-methoxy-phenoxy)-propyl)-amino)-propin-1-yl]-6,7,8,9-tetrahydro-5H-benzocyclohepten,
7-Hydroxy-7-[3-(N-methyl-N-(2-(3-benzyloxy-phenyl)-ethyl)-amino)-propin-1-yl]-6,7,8,9-tetrahydro-5H-benzocyclohepten,
7-Hydroxy-7-[3-(N-methyl-N-(2-(3-benzyloxy-phenyl)-ethyl)-amino)-propen-1-yl]-6,7,8,9-tetrahydro-5H-benzocyclohepten,
7-Hydroxy-7-[3-(N-methyl-N-(2-phenylethyl)-amino)-propin-1-yl]-6,7,8,9-tetrahydro-5H-benzocyclohepten,
7-Hydroxy-7-[3-(N-methyl-N-(3-phenylpropyl)-amino)-propin-1-yl]-6,7,8,9-tetrahydro-5H-benzocyclohepten,
7-Hydroxy-7-[3-(N-methyl-N-(2-(3-methyl-phenyl)-ethyl)-amino)-propin-1-yl]-6,7,8,9-tetrahydro-5H-benzocyclohepten,
7-Hydroxy-7-[3-(N-methyl-N-(2-(3-methoxy-phenyl)-ethyl)-amino)-propin-1-yl]-6,7,8,9-tetrahydro-5H-benzocyclohepten,
7-Methoxy-7-[3-(N-allyl-N-(2-(3,4-dimethoxy-phenyl)-ethyl)-amino)-propin-1-yl]-6,7,8,9-tetrahydro-5H-benzocyclohepten,
7-Hydroxy-7-[3-(N-allyl-N-(2-(3,4-dimethoxy-phenyl)-ethyl)-amino)-propin-1-yl]-6,7,8,9-tetrahydro-5H-benzocyclohepten,
2,3-Dimethoxy-7-hydroxy-7-[3-(N-(3-(3,4-methylendioxy-phenoxy)-propyl)-amino)-propyl]-6,7,8,9-tetrahydro-5H-benzocyclohepten,
2,3-Dimethoxy-7-hydroxy-7-[3-(N-(2-(4-fluor-phenyl)-ethyl)-amino)-propyl]-6,7,8,9-tetrahydro-5H-benzocyclohepten,

2,3-Dimethoxy-7-hydroxy-7-[3-(N-(2-(3-methoxy-phenyl)-ethyl)-amino)-propyl]-6,7,8,9-tetrahydro-5H-benzocyclohepten,
2,3-Dimethoxy-7-hydroxy-7-[3-(N-(3-(3,4-dimethyl-phenoxy)-propyl)-amino)-propyl]-6,7,8,9-tetrahydro-5H-benzocyclohepten,
2,3-Dimethoxy-7-hydroxy-7-[3-(N-(3-(3-methoxy-phenoxy)-propyl)-amino)-propyl]-6,7,8,9-tetrahydro-5H-benzocyclohepten,
2,3-Dimethoxy-7-hydroxy-7-[3-(N-(2-(3-hydroxy-phenyl)-ethyl)-amino)-propyl]-6,7,8,9-tetrahydro-5H-benzocyclohepten,
2,3-Dimethoxy-7-hydroxy-7-[3-(N-(2-(3-methansulfonyl-oxy-phenyl)-ethyl)-amino)-propyl]-6,7,8,9-tetrahydro-5H-benzocyclohepten,
2,3-Dimethoxy-7-hydroxy-7-[3-(N-(2-phenylethyl)-amino)-propyl]-6,7,8,9-tetrahydro-5H-benzocyclohepten,
2,3-Dimethoxy-7-hydroxy-7-[3-(N-(3-phenylpropyl)-amino)-propyl]-6,7,8,9-tetrahydro-5H-benzocyclohepten,
2,3-Dimethoxy-7-hydroxy-7-[3-(N-(2-(3-methyl-phenyl)-ethyl)-amino)-propyl]-6,7,8,9-tetrahydro-5H-benzocyclohepten,
2,3-Dimethoxy-7-hydroxy-7-[3-(N-(2-(3-methansulfonylaminophenyl)-ethyl)-amino)-propyl]-6,7,8,9-tetrahydro-5H-benzocyclohepten,
2,3-Dimethoxy-7-hydroxy-7-[3-(N-(2-phenylethyl)-amino)-propyl]-6,7,8,9-tetrahydro-5H-benzocyclohepten,
2,3-Dimethoxy-7-hydroxy-7-[3-(N-(3-phenylpropyl)-amino)-propyl]-6,7,8,9-tetrahydro-5H-benzocyclohepten,
2,3-Dimethoxy-7-hydroxy-7-[3-(N-(2-(3-chlor-phenyl)-ethyl)-amino)-propyl]-6,7,8,9-tetrahydro-5H-benzocyclohepten,
2,3-Dimethoxy-7-hydroxy-7-[3-(N-(2-(3-acetylamino-phenyl)-ethyl)-amino)-propyl]-6,7,8,9-tetrahydro-5H-benzocyclohepten,
2,3-Dimethoxy-7-hydroxy-7-[3-(N-(2-(3-amino-phenyl)-ethyl)-amino)-propyl]-6,7,8,9-tetrahydro-5H-benzocyclohepten,
2,3-Dimethoxy-7-hydroxy-7-[3-(N-methyl-N-(3-(3,4-methylendioxy-phenoxy)-propyl)-amino)-propyl]-6,7,8,9-tetrahydro-5H-benzocyclohepten,
2,3-Dimethoxy-7-hydroxy-7-[3-(N-methyl-N-(2-(4-fluor-phenyl)-ethyl)-amino)-propyl]-6,7,8,9-tetrahydro-5H-benzocyclohepten,
2,3-Dimethoxy-7-hydroxy-7-[3-(N-methyl-N-(2-(3-methoxy-phenyl)-ethyl)-amino)-propyl]-6,7,8,9-tetrahydro-5H-benzocyclohepten,
2,3-Dimethoxy-7-hydroxy-7-[3-(N-methyl-N-(3-(3,4-dimethyl-phenoxy)-propyl)-amino)-propyl]-6,7,8,9-tetrahydro-5H-benzocyclohepten,
2,3-Dimethoxy-7-hydroxy-7-[3-(N-methyl-N-(3-(3-methoxy-phenoxy)-propyl)-amino)-propyl]-6,7,8,9-tetrahydro-5H-benzocyclohepten,
2,3-Dimethoxy-7-hydroxy-7-[3-(N-methyl-N-(2-(3-hydroxy-phenyl)-ethyl)-amino)-propyl]-6,7,8,9-tetrahydro-5H-benzocyclohepten,
2,3-Dimethoxy-7-hydroxy-7-[3-(N-methyl-N-(2-(3-methansulfonyl-oxy-phenyl)-ethyl)-amino)-propyl]-6,7,8,9-tetrahydro-5H-benzocyclohepten,
2,3-Dimethoxy-7-hydroxy-7-[3-(N-methyl-N-(2-phenylethyl)-amino)-propyl]-6,7,8,9-tetrahydro-5H-benzocyclohepten,
2,3-Dimethoxy-7-hydroxy-7-[3-(N-methyl-N-(3-phenylpropyl)-amino)-propyl]-6,7,8,9-tetrahydro-5H-benzocyclohepten,
2,3-Dimethoxy-7-hydroxy-7-[3-(N-methyl-N-(2-(3-methyl-phenyl)-ethyl)-amino)-propyl]-6,7,8,9-tetrahydro-5H-benzocyclohepten,
2,3-Dimethoxy-7-hydroxy-7-[3-(N-methyl-N-(2-(3-methansulfonylamino-phenyl)-ethyl)-amino)-propyl]-6,7,8,9-tetrahydro-5H-benzocyclohepten,
2,3-Dimethoxy-7-hydroxy-7-[3-(N-methyl-N-(2-phenylethyl)-amino)-propyl]-6,7,8,9-tetrahydro-5H-benzocyclohepten,
2,3-Dimethoxy-7-hydroxy-7-[3-(N-methyl-N-(3-phenylpropyl)-amino)-propyl]-6,7,8,9-tetrahydro-5H-benzocyclohepten,
2,3-Dimethoxy-7-hydroxy-7-[3-(N-methyl-N-(2-(3-chlor-phenyl)-ethyl)-amino)-propyl]-6,7,8,9-tetrahydro-5H-benzocyclohepten,
2,3-Dimethoxy-7-hydroxy-7-[3-(N-methyl-N-(2-(3-acetylamino-phenyl)-ethyl)-amino)-propyl]-6,7,8,9-tetrahydro-5H-benzocyclohepten,
2,3-Dimethoxy-7-hydroxy-7-[3-(N-methyl-N-(2-(3-amino-phenyl)-ethyl)-amino)-propyl]-6,7,8,9-tetrahydro-5H-benzocyclohepten,
2,3-Dimethoxy-7-hydroxy-7-[3-(N-ethyl-N-(3-(3,4-methylendioxy-phenoxy)-propyl)-amino)-propyl]-6,7,8,9-tetrahydro-5H-benzocyclohepten,

2,3-Dimethoxy-7-hydroxy-7-[3-(N-ethyl-N-(2-(3-methoxy-phenyl)-ethyl)-amino)-propyl]-6,7,8,9-tetrahydro-5H-benzocyclohepten,
2,3-Dimethoxy-7-hydroxy-7-[3-(N-allyl-N-(2-(3-methoxy-phenyl)-ethyl)-amino)-propyl]-6,7,8,9-tetrahydro-5H-benzocyclohepten,
2,3-Dimethoxy-7-hydroxy-7-[3-(N-allyl-N-(3-(3,4-dimethyl-phenoxy-propyl)-amino)-propyl]-6,7,8,9-tetrahydro-5H-benzocyclohepten,
2,3-Dimethoxy-7-hydroxy-7-[3-(N-allyl-N-(3-(3-methoxy-phenoxy)-propyl)-amino)-propyl]-6,7,8,9-tetrahydro-5H-benzocyclohepten,
7-Hydroxy-7-[3-(N-(3-(3,4-methoxy-phenoxy)-propyl)-amino)-propyl]-6,7,8,9-tetrahydro-5H-benzocyclohepten,
7-Hydroxy-7-[3-(N-(2-(4-fluor-phenyl)-ethyl)-amino)-propyl]-6,7,8,9-tetrahydro-5H-benzocyclohepten,
7-Hydroxy-7-[3-(N-(2-(3-methoxy-phenyl)-ethyl)-amino)-propyl]-6,7,8,9-tetrahydro-5H-benzocyclohepten,
2,3-Dimethoxy-7-hydroxy-7-[4-(N-(3-(3,4-methylendioxy-phenoxy)-propyl)-amino)-butyl]-6,7,8,9-tetrahydro-5H-benzocyclohepten,
2,3-Dimethoxy-7-hydroxy-7-[4-(N-(3-phenylpropyl)-amino)-butyl]-6,7,8,9-tetrahydro-5H-benzocyclohepten,
2,3-Dimethoxy-7-hydroxy-7-[5-(N-(2-(3-methoxy-phenyl)-ethyl)-amino)-pentyl]-6,7,8,9-tetrahydro-5H-benzocyclohepten,
2,3-Dimethoxy-7-hydroxy-7-[5-(N-(3-phenylpropyl)-amino)-pentyl]-6,7,8,9-tetrahydro-5H-benzocyclohepten,
2,3-Dimethoxy-7-hydroxy-7-[5-(N-methyl-N-(3-(3-methoxy-phenoxy)-propyl)-amino)-pentyl]-6,7,8,9-tetrahydro-5H-benzocyclohepten,
2,3-Dimethoxy-7-[4-(N-(3-(3,4-dimethoxy-phenyl)-propyl)-amino)-butyl]-6,7,8,9-tetrahydro-5-H-benzocyclohepten,
2,3-Dimethoxy-7-[3-(N-(3-(3,4-methylendioxy-phenoxy)-propyl)-amino)-propyl]-6,7,8,9-tetrahydro-5H-benzocyclohepten-6-on,
2,3-Dimethoxy-7-[3-(N-(2-(4-fluor-phenyl)-ethyl)-amino)-propyl]-6,7,8,9-tetrahydro-5H-benzocyclohepten-6-on,
2,3-Dimethoxy-7-[3-(N-(2-(3-methoxy-phenyl)-ethyl)-amino)-propyl]-6,7,8,9-tetrahydro-5H-benzocyclohepten-6-on,
2,3-Dimethoxy-7-[3-(N-(3-(3,4-dimethyl-phenoxy)-propyl)-amino)-propyl]-6,7,8,9-tetrahydro-5H-benzocyclohepten-6-on,
2,3-Dimethoxy-7-[3-(N-(3-(3-methoxy-phenoxy)-propyl)-amino)-propyl]-6,7,8,9-tetrahydro-5H-benzocyclohepten-6-on,
2,3-Dimethoxy-7-[3-(N-(2-(3-hydroxy-phenyl)-ethyl)-amino)-propyl]-6,7,8,9-tetrahydro-5H-benzocyclohepten-6-on,
2,3-Dimethoxy-7-[3-(N-(2-(3-methansulfonyl-oxy-phenyl)-ethyl)-amino)-propyl]-6,7,8,9-tetrahydro-5H-benzocyclohepten-6-on,
2,3-Dimethoxy-7-[3-(N-(2-phenylethyl)-amino)-propyl]-6,7,8,9-tetrahydro-5H-benzocyclohepten-6-on,
2,3-Dimethoxy-7-[3-(N-(3-phenylpropyl)-amino)-propyl]-6,7,8,9-tetrahydro-5H-benzocyclohepten-6-on,
2,3-Dimethoxy-7-[3-(N-(2-(3-methyl-phenyl)-ethyl)-amino)-propyl]-6,7,8,9-tetrahydro-5H-benzocyclohepten-6-on,
2,3-Dimethoxy-7-[3-(N-(2-(3-methansulfonylamino-phenyl)-ethyl)-amino)-propyl]-6,7,8,9-tetrahydro-5H-benzocyclohepten-6-on,
2,3-Dimethoxy-7-[3-(N-(2-phenylethyl)-amino)-propyl]-6,7,8,9-tetrahydro-5H-benzocyclohepten-6-on,
2,3-Dimethoxy-7-[3-(N-(3-phenylpropyl)-amino)-propyl]-6,7,8,9-tetrahydro-5H-benzocyclohepten-6-on,
2,3-Dimethoxy-7-[3-(N-(2-(3-chlor-phenyl)-ethyl)-amino)-propyl]-6,7,8,9-tetrahydro-5H-benzocyclohepten-6-on,
2,3-Dimethoxy-7-[3-(N-(2-(3-acetylamino-phenyl)-ethyl)-amino)-propyl]-6,7,8,9-tetrahydro-5H-benzocyclohepten-6-on,
2,3-Dimethoxy-7-[3-(N-(2-(3-amino-phenyl)-ethyl)-amino)-propyl]-6,7,8,9-tetrahydro-5H-benzocyclohepten-6-on,
2,3-Dimethoxy-7-[3-(N-methyl-N-(3-(3,4-methylendioxy-phenoxy)-propyl)-amino)-propyl]-6,7,8,9-tetrahydro-5H-benzocyclohepten-6-on,
2,3-Dimethoxy-7-[3-(N-methyl-N-(2-(4-fluor-phenyl)-ethyl)-amino)-propyl]-6,7,8,9-tetrahydro-5H-benzocyclohepten-6-on,
2,3-Dimethoxy-7-[3-(N-methyl-N-(2-(3-methoxy-phenyl)-ethyl)-amino)-propyl]-6,7,8,9-tetrahydro-5H-benzocyclohepten-6-on,
2,3-Dimethoxy-7-[3-(N-methyl-N-(3-(3,4-dimethyl-phenoxy)-propyl)-amino)-propyl]-6,7,8,9-tetrahydro-5H-benzocyclohepten-6-on,

2,3-Dimethoxy-7-[3-(N-methyl-N-(3-(3-methoxy-phenoxy)-propyl)-amino)-propyl]-6,7,8,9-tetrahydro-5H-benzocyclohepten-6-on,
2,3-Dimethoxy-7-[3-(N-methyl-N-(2-(3-hydroxy-phenyl)-ethyl)-amino)-propyl]-6,7,8,9-tetrahydro-5H-benzocyclohepten-6-on,
2,3-Dimethoxy-7-[3-(N-methyl-N-(2-(3-methansulfonyl-oxy-phenyl)-ethyl)-amino)-propyl]-6,7,8,9-tetrahydro-5H-benzocyclohepten-6-on,
2,3-Dimethoxy-7-[3-(N-methyl-N-(2-phenylethyl)-amino)-propyl]-6,7,8,9-tetrahydro-5H-benzocyclohepten-6-on,
2,3-Dimethoxy-7-[3-(N-methyl-N-(3-phenylpropyl)-amino)-propyl]-6,7,8,9-tetrahydro-5H-benzocyclohepten-6-on,
2,3-Dimethoxy-7-[3-(N-methyl-N-(2-(3-methyl-phenyl)-ethyl)-amino)-propyl]-6,7,8,9-tetrahydro-5H-benzocyclohepten-6-on,
2,3-Dimethoxy-7-[3-(N-methyl-N-(2-(3-methansulfonylamino-phenyl)-ethyl)-amino)-propyl]-6,7,8,9-tetrahydro-5H-benzocyclohepten-6-on,
2,3-Dimethoxy-7-[3-(N-methyl-N-(2-phenylethyl)-amino)-propyl]-6,7,8,9-tetrahydro-5H-benzocyclohepten-6-on,
2,3-Dimethoxy-7-[3-(N-methyl-N-(3-phenylpropyl)-amino)-propyl]-6,7,8,9-tetrahydro-5H-benzocyclohepten-6-on,
2,3-Dimethoxy-7-[3-(N-methyl-N-(2-(3-chlor-phenyl)-ethyl)-amino)-propyl]-6,7,8,9-tetrahydro-5H-benzocyclohepten-6-on,
2,3-Dimethoxy-7-[3-(N-methyl-N-(2-(3-acetylamino-phenyl)-ethyl)-amino)-propyl]-6,7,8,9-tetrahydro-5H-benzocyclohepten-6-on,
2,3-Dimethoxy-7-[3-(N-methyl-N-(2-(3-amino-phenyl)-ethyl)-amino)-propyl]-6,7,8,9-tetrahydro-5H-benzocyclohepten-6-on,
2,3-Dimethoxy-7-[3-(N-ethyl-N-(3-(3,4-methylendioxy-phenoxy)-propyl)-amino)-propyl]-6,7,8,9-tetrahydro-5H-benzocyclohepten-6-on,
2,3-Dimethoxy-7-[3-(N-ethyl-N-(2-(3-methoxy-phenyl)-ethyl)-amino)-propyl]-6,7,8,9-tetrahydro-5H-benzocyclohepten-6-on,
2,3-Dimethoxy-7-[3-(N-allyl-N-(2-(3-methoxy-phenyl)-ethyl)-amino)-propyl]-6,7,8,9-tetrahydro-5H-benzocyclohepten-6-on,
2,3-Dimethoxy-7-[3-(N-allyl-N-(3-(3,4-dimethyl-phenoxy)-propyl)-amino)-propyl]-6,7,8,9-tetrahydro-5H-benzocyclohepten-6-on,
2,3-Dimethoxy-7-[3-(N-allyl-N-(3-(3-methoxy-phenoxy)-propyl)-amino)-propyl]-6,7,8,9-tetrahydro-5H-benzocyclohepten-6-on,
7-Hydroxy-7-[3-(N-(3-(3,4-methylendioxy-phenoxy)-propyl)-amino)-propyl]-6,7,8,9-tetrahydro-5H-benzocyclohepten-6-on,
7-Hydroxy-7-[3-(N-(2-(4-fluor-phenyl)-ethyl)-amino)-propyl]-6,7,8,9-tetrahydro-5H-benzocyclohepten-6-on,
7-Hydroxy-7-[3-(N-(2-(3-methoxy-phenyl)-ethyl)-amino)-propyl]-6,7,8,9-tetrahydro-5H-benzocyclohepten-6-on und
2,3-Dimethoxy-7-[4-(N-(3-(3,4-methylendioxy-phenoxy)-propyl)-amino)-butyl]-6,7,8,9-tetrahydro-5H-benzocyclohepten-6-on,
deren Enantiomere, deren Diastereomere und deren Säureadditionssalze.

Bevorzugte Verbindungen der obigen Formel I sind jedoch diejenigen, in denen
$X_1$ ein Wasserstoffatom, $X_2$ ein Wasserstoffatom und $X_3$ ein Wasserstoffatom, eine Hydroxy- oder Methoxygruppe oder
$X_1$ und $X_3$ zusammen eine weitere Kohlenstoff-Kohlenstoff-Bindung und $X_2$ ein Wasserstoffatom oder
$X_1$ und $X_2$ zusammen mit dem dazwischenliegenden Kohlenstoffatom eine Carbonylgruppe und $X_3$ ein Wasserstoffatom,
$A_1$ eine n-Propylengruppe, in welche die mit dem Cycloheptenring verknüpfte Ethylengruppe durch eine Ethenylen- oder Ethinylengruppe ersetzt sein kann,
$A_2$ eine Ethylen- oder n-Propylengruppe oder eine n-Propylengruppe, in der, falls n die Zahl 0 darstellt, eine im Rest $A_2$ enthaltene Ethylengruppe, welche mit dem Rest $R_4$ verknüpft ist, durch eine Ethenylengruppe ersetzt ist,
$R_1$ ein Wasserstoffatom, eine Methyl- oder Methoxygruppe,
$R_2$ ein Wasserstoffatom, eine Methyl- oder eine Methoxygruppe,
$R_3$ die Methylgruppe,
n die Zahl 0 oder 1,
$R_5$ ein Wasserstoff-, Fluor-, Chlor- oder Bromatom, eine Hydroxy-, Methoxy-, Cyano-, Methyl-, Nitro-,

Amino-, Methylsulfonyloxy-, Trifluormethylsulfonyloxy- oder Benzyloxygruppe,
$R_6$ ein Wasserstoff-, Chlor- oder Bromatom oder eine Methoxygruppe und
$R_7$ ein Wasserstoff-, Chlor- oder Bromatom bedeuten, deren Enantiomere, deren Diastereomere und deren Säureadditionssalze.

Besonders bevorzugte Verbindungen der Formel I sind diejenigen, in denen
$X_1$ ein Wasserstoffatom, $X_2$ ein Wasserstoffatom und $X_3$ ein Wasserstoffatom oder eine Hydroxygruppe oder
$X_1$ und $X_3$ zusammen eine weitere Kohlenstoff-Kohlenstoff-Bindung und $X_2$ ein Wasserstoffatom oder
$X_1$ und $X_2$ zusammen mit dem dazwischenliegenden Kohlenstoffatom eine Carbonylgruppe und $X_3$ ein Wasserstoffatom,
$R_1$ eine Methoxygruppe,
$R_2$ eine Methoxygruppe,
$R_3$ eine Methylgruppe,
$A_1$ eine n-Propylengruppe,
$A_2$ eine Ethylen- oder n-Propylengruppe,
$R_5$ eine Methoxy- oder Methylsulfonyloxygruppe,
$R_6$ ein Wasserstoffatom oder eine Methoxygruppe,
$R_7$ ein Wasserstoffatom und
n die Zahl 0 oder 1 bedeuten, deren Enantiomere, deren Diastereomere und deren Säureadditionssalze.

Erfindungsgemäß erhält man die neuen Verbindungen nach folgenden Verfahren:

a) Umsetzung einer Verbindung der Formel

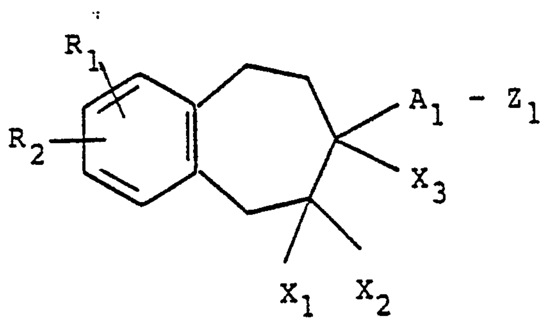

,(II)

mit einer Verbindung der Formel

$Z_2 - A_2 - R_4$ ,(III)

in denen
$R_1$, $R_2$, $R_4$, $A_1$, $A_2$ und $X_1$ bis $X_3$ wie eingangs definiert sind,
einer der Reste $Z_1$ oder $Z_2$ eine $R_3$-NH-Gruppe, wobei $R_3$ wie eingangs definiert ist, und
der andere der Reste $Z_1$ oder $Z_2$ eine nukleophile Austrittsgruppe wie ein Halogenatom oder eine Sulfonyloxygruppe darstellt, z.B. ein Chlor-, Brom- oder Jodatom, die Methansulfonyloxy-, p-Toluolsulfonyloxy- oder Ethoxysulfonyloxygruppe.

Die Umsetzung wird zweckmäßigerweise in einem Lösungsmittel oder Lösungsmittelgemisch wie Aceton, Diethylether, Methylformamid, Dimethylformamid, Dimethylsulfoxid, Benzol, Chlorbenzol, Tetrahydrofuran, Benzol/Tetrahydrofuran, Dioxan oder in einem Überschuß der eingesetzten Verbindungen der Formeln II und/oder III und gegebenenfalls in Gegenwart eines säurebindenden Mittels, z.B. eines Alkoholats wie Kalium-tert.butylat, eines Alkalihydroxids wie Natrium- oder Kaliumhydroxid, eines Alkalicarbonats wie Kaliumcarbonat, eines Alkaliamids wie Natriumamid, eines Alkalihydrids wie Natriumhydrid, einer tertiären organischen Base wie Triethylamin oder Pyridin, wobei die letzteren gleichzeitig auch als Lösungsmittel dienen können, oder eines Reaktionsbeschleunigers wie Kaliumjodid je nach der Reaktionsfähigkeit des nukleophil austauschbaren Restes zweckmäßigerweise bei Temperaturen zwischen 0 und 150 °C, vorzugsweise bei Temperaturen zwischen 50 und 120 °C, z.B. bei der Siedetemperatur des verwendeten Lösungsmittels, durchgeführt. Die Umsetzung kann jedoch auch ohne Lösungsmittel durchgeführt werden. Besonders vorteilhaft wird die Umsetzung jedoch in Gegenwart einer tertiären organischen Base oder eines Überschusses eines eingesetzten Amins der Formel II oder III durchgeführt.

b) Zur Herstellung von Verbindungen der Formel I, in der $X_3$ ein Wasserstoffatom darstellt:
Katalytische Hydrierung einer Verbindung der Formel

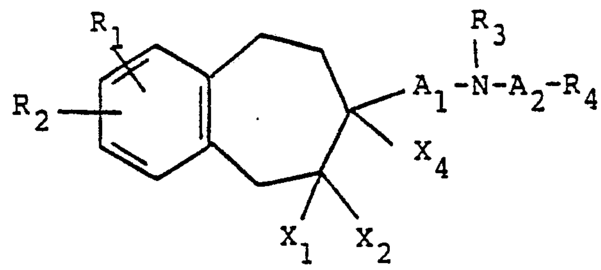

,(IV)

in der
$R_1$ bis $R_4$, $X_1$, $X_2$, $A_1$ und $A_2$ wie eingangs definiert sind, wobei jedoch
$X_4$ eine Hydroxygruppe oder
$X_1$ und $X_4$ zusammen eine weitere Kohlenstoff-Kohlenstoff-Bindung oder
$X_4$ zusammen mit einem Wasserstoffatom des benachbarten gesättigten Kohlenstoffatoms des Restes $A_1$ eine Kohlenstoff-Kohlenstoff-Bindung darstellen muß.

Die katalytische Hydrierung wird vorzugsweise in einem Lösungsmittel wie Methanol, Ethanol, Eisessig, Essigsäurethylester oder Dimethylformamid mit Wasserstoff bei einem Wasserstoffdruck von 1 bis 5 bar, vorzugsweise von 1 bis 4 bar, in Gegenwart eines Hydrierungskatalysators wie Platin, Palladium/Kohle oder Raney-Nickel, gegebenenfalls in Gegenwart einer Säure wie Perchlorsäure bei Temperaturen zwischen 0 und 80 °C, vorzugsweise jedoch bei Temperaturen zwischen Raumtemperatur und 60 °C, durchgeführt.

Bedeutet $X_4$ eine Hydroxygruppe so wird die katalytische Hydrierung in Gegenwart einer Säure durchgeführt.

Bei der katalytischen Hydrierung können vorhandene Doppel-oder Dreifachbindungen gleichzeitig aufhydriert oder gegebenenfalls vorhandene Benzylgruppen abgespalten werden.

c) Zur Herstellung von Verbindungen der Formel I, in der $X_1$ und $X_3$ eine weitere Kohlenstoff-Kohlenstoff-Bindung darstellen:
Abspaltung eines Restes $HZ_3$ von einer Verbindung der Formel

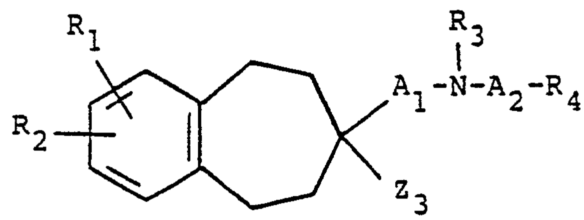

,(V)

in der
$R_1$ bis $R_4$, $A_1$ und $A_2$ wie eingangs definiert sind und
$Z_3$ eine abspaltbare Gruppe wie eine Hydroxy-, Alkoxy-, Acyloxy- oder Alkylsulfonyloxygruppe darstellt.

Die Umsetzung wird vorzugsweise in einem Lösungsmittel wie Ethanol, Isopropanol, Tetrahydrofuran, Dioxan oder Pyridin gegebenfalls in Gegenwart eines säurebindenden Mittels wie Natriumcarbonat oder Pyridin oder in Gegenwart einer Säure wie Salzsäure oder Schwefelsäure bei Temperaturen zwischen 0 und 100 °C, vorzugsweise bei Temperaturen zwischen 20 und 80 °C, durchgeführt.

Ein gegebenenfalls so erhaltenes Isomerengemisch bestehend aus einer Verbindung der Formel I, in der $X_1$ und $X_3$ zusammen eine weitere Kohlenstoff-Kohlenstoff-Bindung darstellen, und einer Verbindung der Formel I, in der $X_3$ zusammen mit einem Wasserstoffatom des benachbarten gesättigten Kohlenstoffatoms des Restes $A_1$ eine Kohlenstoff-Kohlenstoff-Bindung darstellt, wird anschließend mittels Chromatographie, z. B. über Aluminiumoxid (neutral), aufgetrennt.

d) Zur Herstellung von Verbindungen der Formel I, in der $X_3$ die Hydroxygruppe darstellt:
Umsetzung einer Verbindung der Formel

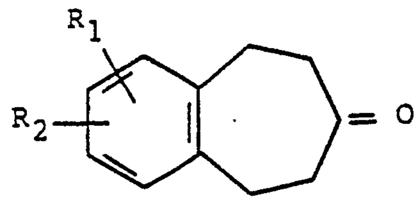

,(VI),

in der
$R_1$ und $R_2$ wie eingangs definiert sind, mit einer Verbindung der Formel

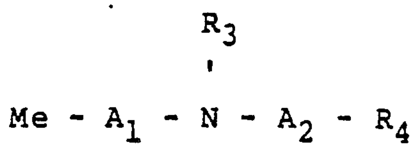

,(VII)

in der
$R_3$, $R_4$, $A_1$ und $A_2$ wie eingangs definiert sind und
Me ein Alkaliatom oder eine MgHal-Gruppe darstellt, wobei Hal ein Chlor-, Brom- oder Jodatom bedeutet.
Die Umsetzung wird vorzugsweise in einem Lösungsmittel wie Diethylether, Methyl-tert.butylether, Tetrahydrofuran, Dioxan, n-Hexan oder Benzol gegebenenfalls unter Schutzgas wie Stickstoff oder Argon bei Temperaturen zwischen 0 und 50° C, vorzugsweise jedoch bei Raumtemperatur, durchgeführt.

e) Zur Herstellung von Verbindungen der Formel I, in der $R_5$ eine Alkylamino-, Dialkylamino-, Alkanoylamino-, Alkoxycarbonylamino-, Alkylsulfonylamino-, Bis(alkylsulfonyl)-amino, N-Alkyl-alkylsulfonylamino-, Alkylmercapto-, Alkoxy-, Alkoxycarbonyloxy-, Hydroxycarbonylalkoxy, Alkoxycarbonylalkoxy-, Phenylalkoxy-, Trifluormethoxy-, Difluormethoxy-, Cyanoalkoxy-, Alkylsulfonyloxy- oder Trifluormethylsulfonyloxygruppe darstellt:
Umsetzung einer Verbindung der Formel

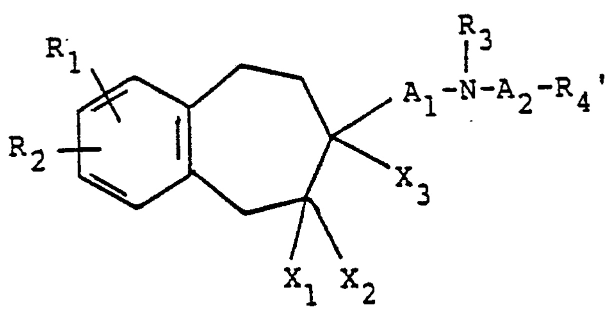

,(VIII)

in der
$R_1$ bis $R_3$, $A_1$, $A_2$ und $X_1$ bis $X_3$ wie eingangs definiert sind und
$R_4'$ eine Gruppe der Formel

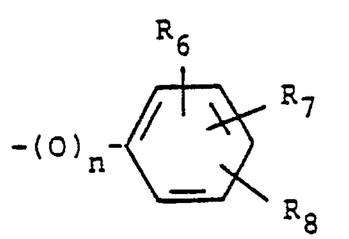

darstellt, wobei

$R_5$, $R_7$ und n wie eingangs definiert sind und
$R_8$ eine Hydroxy-, Amino- oder Alkylaminogruppe mit 1 bis 3 Kohlenstoffatomen darstellt, mit einer

Verbindung der Formel

$Z_4 - R_9$ ,(IX)

in der

$Z_4$ eine nukleophile Austrittsgruppe wie ein Halogenatom, z.B. ein Chlor-, Brom- oder Jodatom, und

$R_9$ eine Alkyl-, Alkanoyl-, Alkoxycarbonyl-, Hydroxycarbonylalkyl-, Alkoxycarbonylalkyl-, Alkylsulfonyl-, Phenylalkyl-, Trifluormethyl-, Difluormethyl- oder Cyanoalkylgruppe bedeuten, wobei die vorstehend erwähnten Alkyl- und Alkoxyteile jeweils 1 bis 3 Kohlenstoffatome bzw. der Alkanoylteil 2 oder 3 Kohlenstoffatome enthalten kann.

Die Umsetzung wird zweckmäßigerweise in einem Lösungsmittel oder Lösungsmittelgemisch wie Methylenchlorid, Chloroform, Tetrachlorkohlenstoff, Ether, Tetrahydrofuran, Dioxan, Benzol, Toluol, Acetonitril oder Dimethylformamid, gegebenenfalls auch in Gegenwart eines die Säure aktivierenden Mittels oder eines wasserentziehenden Mittels, z. B. in Gegenwart von Chlorameisensäureethylester, Thionylchlorid, Phosphortrichlorid, Phosphorpentoxid, N,N′-Dicyclohexylcarbodiimid, N,N′-Dicyclohexylcarbodiimid/N-Hydroxysuccinimid, N,N′-Carbonyldiimidazol oder N,N′-Thionyldiimidazol, und gegebenenfalls in Gegenwart einer anorganischen Base wie Natriumcarbonat oder einer tertiären organischen Base wie Triethylamin oder Pyridin, wobei die beiden letzteren auch gleichzeitig als Lösungsmittel dienen können, bei Temperaturen zwischen -25 und 250 °C, vorzugsweise jedoch bei Temperaturen zwischen -10 °C und der Siedetemperatur des verwendeten Lösungsmittels, durchgeführt werden.

Bedeutet $R_3$ in einer Verbindung der Formel VIII ein Wasserstoffatom, so wird dieses, falls es nicht während der Umsetzung durch einen üblichen Schutzrest geschützt ist, gleichzeitig durch einen entsprechenden $R_9$ ersetzt.

f) Reduktion einer Verbindung der Formel

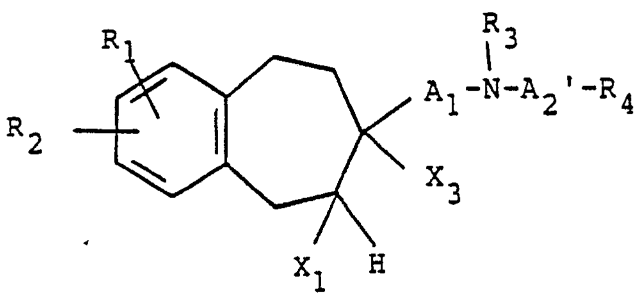

,(X)

in der

$R_1$ bis $R_4$, $A_1$, $X_1$ und $X_3$ wie eingangs definiert sind und

$A_2$, eine gegebenenfalls durch eine Alkylgruppe mit 1 bis 3 Kohlenstoffatomen substituierte geradkettige Alkylengruppe mit 2 bis 4 Kohlenstoffatomen darstellt, in welcher eine mit dem Stickstoffatom der $R_3$-N< Gruppe verknüpfte Methylengruppe durch eine Carbonylgruppe ersetzt ist.

Die Reduktion wird vorzugsweise mit einem Metallhydrid wie Lithiumaluminiumhydrid oder Diboran oder mit einem Komplex aus Boran und einem Thioäther, z.B. mit Boran-Dimethylsulfid-Komplex, in einem geeigneten Lösungsmittel wie Diäthyläther oder Tetrahydrofuran bei Temperaturen zwischen 0 und 80 °C, vorzugsweise jedoch bei Temperaturen zwischen 10 und 45 °C, durchgeführt.

g) Zur Herstellung von Verbindungen der Formel I, in der $A_1$ eine gegebenenfalls durch eine Alkylgruppe mit 1 bis 3 Kohlenstoffatomen substituierte geradkettige Alkylengruppe mit 3 oder 4 Kohlenstoffatomen darstellt, in welcher eine mit dem Benzocycloheptenring verknüpfte Ethylengruppe durch eine Ethenylengruppe ersetzt sein kann:

Katalytische Hydrierung einer Verbindung der Formel

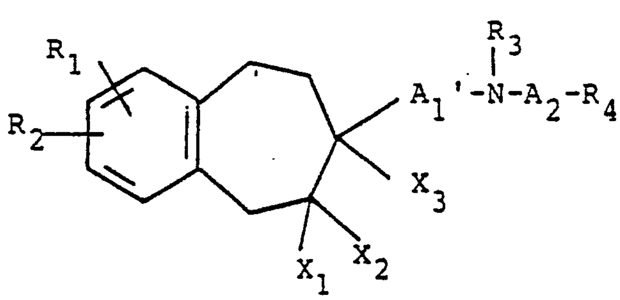

,(XI)

in der
$R_1$ bis $R_4$, $A_2$ und $X_1$ bis $X_3$ wie eingangs definiert sind und
$A_1'$ eine gegebenenfalls durch eine Alkylgruppe mit 1 bis 3 Kohlenstoffatomen substituierte geradkettige Alkylengruppe mit 3 oder 4 Kohlenstoffatomen darstellt, in welcher eine im Rest $A_1'$ enthaltene mit dem Benzocycloheptenring verknüpfte Ethylengruppe durch eine Ethenylen- oder Ethinylengruppe ersetzt ist.
Die katalytische Hydrierung wird vorzugsweise in einem Lösungsmittel wie Methanol, Ethanol, Eisessig, Essigsäurethylester oder Dimethylformamid mit Wasserstoff bei einem Wasserstoffdruck von 1 bis 5 bar, vorzugsweise von 1 bis 4 bar, in Gegenwart eines Hydrierungskatalysators wie Platin, Palladium/Kohle oder Raney-Nickel, gegebenenfalls in Gegenwart einer Säure wie Perchlorsäure bei Temperaturen zwischen 0 und 80°C, vorzugsweise jedoch bei Temperaturen zwischen Raumtemperatur und 60°C, durchgeführt.
Zur Herstellung von Verbindungen der Formel I, in der $A_1$ eine Doppelbindung enthält, wird die katalytische Hydrierung einer Verbindung der Formel XI, in der $A_1'$ eine Dreifachbindung enthält, vorzugsweise in Gegenwart von Raney-Nickel oder Lindlar-Katalysator durchgeführt.
Bei der katalytischen Hydrierung kann desweiteren eine im Rest $A_2$ vorhandene Doppelbindung gleichzeitig aufhydriert werden.

Bei den vorstehend beschriebenen Umsetzungen können gegebenenfalls vorhandene reaktive Gruppen wie Hydroxy-, Amino-oder Iminogruppen während der Umsetzung durch übliche Schutzgruppen geschützt werden, welche nach der Umsetzung wieder abgespalten werden.

Beispielsweise kommt als Schutzrest für eine Hydroxygruppe die Trimethylsilyl-, Acetyl-, Benzoyl-, Benzyl- oder Tetrahydropyranylgruppe und
als Schutzrest für eine Imino- oder Aminogruppe die Acetyl-, Benzoyl-, Ethoxycarbonyl- oder Benzylgruppe in Betracht.

Die gegebenenfalls anschließende Abspaltung eines verwendeten Schutzrestes erfolgt vorzugsweise hydrolytisch in einem wässrigen Lösungsmittel, z.B. in Wasser, Isopropanol/Wasser, Tetrahydrofuran/Wasser oder Dioxan/Wasser, in Gegenwart einer Säure wie Salzsäure oder Schwefelsäure oder in Gegenwart einer Alkalibase wie Natriumhydroxid oder Kaliumhydroxid bei Temperaturen zwischen 0 und 100°C, vorzugsweise bei der Siedetemperatur des Reaktionsgemisches. Die Abspaltung eines Benzylrestes erfolgt jedoch vorzugsweise hydrogenolytisch, z.B. mit Wasserstoff in Gegenwart eines Katalysators wie Palladium/Kohle in einem Lösungsmittel wie Metha nol, Ethanol, Essigsäureethylester oder Eisessig gegebenenfalls unter Zusatz einer Säure wie Salzsäure bei Temperaturen zwischen 0 und 50°C, vorzugsweise jedoch bei Raumtemperatur, und einem Wasserstoffdruck von 1 bis 7 bar, vorzugsweise jedoch von 3 bis 5 bar.

Erhält man erfindungsgemäß eine Verbindung der Formel I, in der $R_5$ eine Benzyloxygruppe darstellt, so kann diese mittels Entbenzylierung in die entsprechende Hydroxyverbindung übergeführt werden.

Die nachträgliche Entbenzylierung wird vorzugsweise in einem Lösungsmittel wie Wasser, Wasser/Ethanol, Methanol, Eisessig, Essigsäureethylester oder Dimethylformamid zweckmäßigerweise mit Wasserstoff in Gegenwart eines Hydrierungskatalysators wie Raney-Nickel, Platin oder Palladium/Kohle bei Temperaturen zwischen 0 und 50°C, vorzugsweise jedoch bei Raumtemperatur, durchgeführt.

Die erhaltenen Verbindungen der Formel I lassen sich, sofern diese mindestens ein chirales Zentrum besitzen, mittels üblichen Methoden in ihre Diastereomeren, beispielsweise durch Säulenchromatographie, und in ihre Enantiomeren auftrennen, beispielsweise durch Säulenchromatographie an einer chiralen Phase oder durch Kristallisation mit optisch aktiven Säuren, z.B. mit D- oder L-Monomethylweinsäure, D- oder L-Diacetylweinsäure, D- oder L-Weinsäure, D- oder L-Milchsäure, D- oder L-Camphersäure, D- oder L-Dibenzoylweinsäure, D- oder L-Camphersulfonsäure oder D- oder L-Camphansäure.

Die erhaltenen Verbindungen der Formel I lassen sich ferner in ihre Säureadditionssalze, insbesondere für ihre pharmazeutische Anwendung in ihre physiologisch verträglichen Säureadditionssalze mit anorganischen oder organischen Säuren überführen. Als Säuren kommen hierbei beispielsweise Salzsäure, Bromwasserstoffsäure, Schwefelsäure, Phosphorsäure, Essigsäure, Milchsäure, Zitronensäure, Weinsäure, Bernsteinsäure, Maleinsäure, Fumarsäure oder Oxalsäure in Betracht.

Die als Ausgangsstoffe verwendeten Verbindungen der Formeln II bis XI sind teilweise literaturbekannt bzw. man erhält sie nach an sich bekannten Verfahren.

Beispielsweise erhält man eine Ausgangsverbindung der Formel II durch Umsetzung eines entsprechenden Benzocyclohepten-7-ons mit einer entsprechenden metallorganischen Verbindung und gegebenenfalls anschließende Dehydratisierung und/oder Hydrierung. Eine gegebenenfalls so erhaltene Pyranyl-2-oxy-Verbindung kann anschließend in eine Verbindung der Formel II, in der $Z_1$ ein Halogenatom darstellt, übergeführt werden.

Eine als Ausgangsstoff verwendete Verbindung der Formel IV, V, VIII, X oder XI erhält man durch

Umsetzung eines entsprechenden Benzocycloheptenderivates mit einem entsprechenden Alkylhalogenid oder Alkylamin.

Wie bereits eingangs erwähnt, weisen die neuen Verbindungen der Formel I und deren physiologisch verträgliche Säureadditionssalze mit anorganischen oder organischen Säuren wertvolle pharmakologische Eigenschaften auf, insbesondere bei geringen zentralen Nebenwirkungen eine herzfreguenzsenkende, blutdrucksenkende, $Ca^{++}$ -antagonistische und/oder antithrombotische Wirkung sowie eine antiischämische Wirkung am Herzen und eine den $0_2$-Bedarf des Herzens vermindernde Wirkung.

Beispielsweise wurden die Verbindungen

A = 2,3-Dimethoxy-7-[3-(N-methyl-N-(2-(3,4-dimethoxy-phenyl)-ethyl)-amino)-propyl]-6,7,8,9-tetrahydro-5H-benzocyclohepten,

B = 2,3-Dimethoxy-[3-(N-methyl-N-(2-(4-methoxy-phenyl)-ethyl)-amino)-propyl]-6,7,8,9-tetrahydro-5H-benzocyclohepten,

C = 2,3-Dimethoxy-7-[3-(N-methyl-N-(2-(3-methoxy-phenyl)ethyl)-amino)-propyl]-6,7,8,9-tetrahydro-5H-benzocyclohepten,

D = 2,3-Dimethoxy-7-hydroxy-7-[3-(N-methyl-N-(2-(3-methoxy-phenyl)-ethyl)-amino)-propyl]-6,7,8,9-tetrahydro-5H-benzocyclohepten,

E = 2,3-Dimethoxy-7-hydroxy-7-[3-(N-methyl-N-(3-(3-methoxy-phenoxy)-propyl)-amino)-propyl]-6,7,8,9-tetrahydro-5H-benzocyclohepten und

F = 2,3-Dimethoxy-7-hydroxy-7-[3-(N-methyl-N-(2-(3-methansulfonyloxy-phenyl)-ethyl)-amino)-propyl]-6,7,8,9- tetrahydro-5H-benzo-cyclohepten

auf ihre biologischen Eigenschaften wie folgt untersucht:

Wirkung auf die Herzfrequenz an Ratten:

Die Wirkung der zu untersuchenden Substanzen auf die Herzfrequenz wurde pro Dosis an je 2 Ratten mit einem durchschnittlichen Gewicht von 250-300 g untersucht. Hierzu wurden die Ratten mit Pentobarbital (50 mg/kg i.p. und 10 mg/kg s.c.) narkotisiert. Die zu untersuchenden Substanzen wurden in wäßriger Lösung in die Vena jugularis injiziert (0,1 ml/100 g).

Der Blutdruck wurde über in eine A. carotis eingebundene Kanüle gemessen und die Herzfrequenz wurde aus einem mit Nadelelektroden abgeleiteten EKG (II. oder III. Ableitung) registriert. Die Herzfrequenz der Tiere in der Kontrollperiode lagen zwischen 350 und 400 Schlägen/Minuten (S/min).

Die nachfolgende Tabelle enthält die gefundenen Werte:

| Substanz | Dosis [mg/kg] | Herzfrequenzsenkung, gemessen 5 Minuten nach Substanzapplikation [S/min] |
|---|---|---|
| A | 5,0 | -173 |
| B | 5,0 | -170 |
| C | 5,0 | -180 |
| D | 5,0 | -155 |
| E | 5,0 | -167 |
| F | 5,0 | -130 |

Die erfindungsgemäß hergestellten Verbindungen weisen in therapeutischen Dosen keinerlei toxische Nebenwirkungen auf. So konnten beipielsweise bei einer intravenösen Applikation der Substanz A bis F auch in einer hohen Dosis von 10 mg/kg an Mäusen, außer einer geringen Sedation keine toxischen Nebenwirkungen beobachtet werden.

Aufgrund ihrer pharmakologischen Eigenschaften eignen sich die erfindungsgemäß hergestellen Verbindungen zur Behandlung und Prophylaxe ischämischer Herzerkrankungen, z. B. zur Behandlung und Prophylaxe des Herzinfarktes, und zur Behandlung von Sinustachycardien.

Die zur Erzielung einer entsprechenden Wirkung erforderlichen Dosierung beträgt zweckmäßigerweise ein- bis zweimal täglich 0,03 bis 0,4 mg/kg Körpergewicht, vorzugsweise 0,07 bis 0,25 mg/kg Körpergewicht. Hierzu lassen sich die erfindungsgemäß hergestellten Verbindungen der Formel I sowie ihre physiologisch verträglichen Säureadditionssalze mit anorganischen oder organischen Säuren, gegebenenfalls in Kombination mit anderen Wirksubstanzen, zusammen mit einem oder mehreren inerten üblichen Trägerstoffen und/oder Verdün nungsmitteln, z.B. mit Maisstärke, Milchzucker, Rohrzucker, mikrokristalliner

Zellulose, Magnesiumstearat, Polyvinylpyrrolidon, Zitronensäure, Weinsäure, Wasser, Wasser/ Äthanol, Wasser/Glycerin, Wasser/Sorbit, Wasser/Polyäthylenglykol, Propylenglykol, Carboxymethylcellulose oder fetthaltige Substanzen wie Hartfett oder deren geeignete Gemische, in übliche galenische Zubereitungen wie Tabletten, Dragees, Kapseln, Pulver, Suspensionen, Tropfen, Ampullen, Säfte oder Zäpfchen einarbeiten.

Die nachfolgenden Beispiele sollen die Erfindung näher erläutern:

Beispiel A

N-Benzyl-N-methyl-propargylamin

Eine Lösung von 78,9 ml (0,6 Mol) N-Methyl-benzylamin und 83,6 ml (0,6 Mol) Triethylamin wird in 500 ml Diethylether bei 22° C unter Eiskühlung während 45 Minuten mit 45,4 ml (0,6 Mol) Propargylbromid versetzt. Nach 2 Stunden bei Raumtemperatur wird mit 500 ml Wasser extrahiert, die organische Phase über Magnesiumsulfat getrocknet, im Vakuum eingedampft und destilliert.
Ausbeute: 74,4 g (78 % der Theorie),
$Kp_{15\text{-}20\ mm}$: 108-115° C.

Beispiel B

2,3-Dimethoxy-7-hydroxy-7-(3-(N-benzyl-N-methyl-amino)-propin-1-yl)-6,7,8,9-tetrahydro-5H-benzocyclohepten

Zu einer aus 11,4 g (0,467 Mol) Magnesium in 40 ml Diethylether und 34,3 ml (0,467 Mol) Ethylbromid frisch hergestellten Lösung von Ethylmagnesiumbromid in 80 ml Diethylether werden bei 15° C 170 ml Tetrahydrofuran zugetropft. Anschliessend wird bei 20-37° C eine Lösung von 74,2 g (0,467 Mol) N-Benzyl-N-methyl-propargylamin in 80 ml Tetrahydrofuran zugetropft. Nach beendeter Ethanentwicklung tropft man 82,2 g (0,373 Mol) 2,3-Dimethoxy-6,7,8,9-tetrahydro-5H-benzocyclohepten-7-on in 160 ml Tetrahydrofuran zu. Nach 30 Minuten bei ca. 30° C wird mit 100 ml 10%iger Ammoniumchlorid-Lösung versetzt, mit Diethylether extrahiert, mit halbgesättigter Kaliumcarbonat-Lösung und mit Wasser gewaschen. Die organische Phase wird abgetrennt, über Magnesiumsulfat getrocknet und im Vakuum eingedampft. Das erhaltene Rohprodukt wird über 3 kg Aluminiumoxid (neutral, Aktivität II-III) mit Methylenchlorid und anschließend mit ansteigenden Anteilen von Ethanol (bis 1 %) gereinigt.
Ausbeute: 127 g (90 % der Theorie),
$R_F$-Wert: 0,2 (Aluminiumoxid, Laufmittel: 2 % Ethanol in Methylenchlorid).

Beispiel C

2,3-Dimethoxy-7-hydroxy-7-(3-(N-benzyl-N-methyl-amino)-propyl)-6,7,8,9-tetrahydro-5H-benzocyclohepten

24,2 g (0,064 Mol) 2,3-Dimethoxy-7-hydroxy-7-(3-(N-benzyl-N-methyl-amino)-propin-1-yl)-6,7,8,9-tetrahydro-5H-benzocyclohepten werden in 300 ml Methanol in Anwesenheit von 4 g Raney-Nickel 20 Stunden bei Raumtemperatur und 5 bar Wasserstoff hydriert. Nach Filtration und Eindampfen im Vakuum wird das Rohprodukt über 1600 g Aluminiumoxid (neutral, Aktivität II-III) mit Methylenchlorid und ansteigenden Anteilen von Ethanol (bis 3 %) gereinigt.
Ausbeute: 24 g (98 % der Theorie),
$R_F$-Wert: 0,65 (Aluminiumoxid, Laufmittel: 3 % Ethanol in Methylenchlorid).

Beispiel D

Isomerengemisch aus 2,3-Dimethoxy-7-(3-(N-benzyl-N-methyl-amino)-propyl)-8,9-dihydro-5H-benzocyclohepten und 2,3-Dimethoxy-7-(3-(N-benzyl-N-methyl-amino)-propyliden)-6,7,8,9-tetrahydro-5H-benzocyclohepten

23,8 g (0,062 Mol) 2,3-Dimethoxy-7-hydroxy-7-(3-(N-benzyl-N-methyl-amino)-propyl)-6,7,8,9-tetrahydro-5H-benzocyclohepten und 5,5 g (0,029 Mol) p-Toluolsulfonsäurehydrat werden in 600 ml Toluol 6 Stunden am Wasserabscheider gekocht. Es wird mit gesättigter Natriumhydrogencarbonat-Lösung extra hiert, die organische Phase über Magnesiumsulfat getrocknet, im Vakuum eingedampft und das Rohprodukt über 1600 g Aluminiumoxid (neutral, Aktivität II-III) mit Methylenchlorid gereinigt.
Ausbeute: 18,3 g (80 % der Theorie),
$R_F$-Wert: 0,87-0,92 (Aluminiumoxid, Laufmittel: Cyclohexan + 50 % Essigester).

Beispiel E

2,3-Dimethoxy-7-(3-(N-methyl-amino)-propyl)-6,7,8,9-tetrahydro-5H-benzocyclohepten

8,3 g (0,0227 Mol) eines Isomerengemisches von 2,3-Dimethoxy-7-(3-(N-benzyl-N-methyl-amino)-propyl)-8,9-dihydro-5H-benzocyclohepten und 2,3-Dimethoxy-7-(3-(N-benzyl-N-methyl-amino)-propyliden)-6,7,8,9-tetrahydro-5H-benzocyclohepten werden in 150 ml Eisessig in Anwesenheit von 1 g 10%igem Palladium auf Aktivkohle 4 Stunden bei 50 °C und 2 bar Wasserstoff hydriert. Das im Vakuum eingedampfte Filtrat wird in Methylenchlorid gelöst und mit gesättigter Natriumhydrogencarbonat-Lösung gewaschen. Die organische Phase wird über Magnesiumsulfät getrocknet, im Vakuum eingedampft und das erhaltene Rohprodukt über Aluminiumoxid (neutral, Aktivität II-III) mit Methylenchlorid und ansteigenden Anteilen von Ethanol (bis 15 %) gereinigt.
Ausbeute: 2,27 g (36,0 % der Theorie),
$R_F$-Wert: 0,25 (Aluminiumoxid, Laufmittel: 10 % Ethanol in Methylenchlorid).

Beispiel F

2,3-Dimethoxy-7-hydroxy-7-(3-(N-methyl-amino)-propyl)-6, 7,8,9-tetrahydro-5H-benzocyclohepten

8,15 g (0,0215 Mol) 2,3-Dimethoxy-7-hydroxy-7-(3-(N-benzyl- N-methyl-amino)-propin-1-yl)-6,7,8,9-tetrahydro-5H-benzocyclohepten werden in 120 ml Ethanol in Anwesenheit von 2 g 10%igem Palladium auf Aktivkohle 7 Stunden bei Raumtemperatur und 5 bar Wasserstoff hydriert. Das im Vakuum eingedampfte Filtrat wird über 500 g Aluminiumoxid (neutral, Aktivität II-III) mit Methylenchlorid und ansteigenden Anteilen von Ethanol (bis 10 %) gereinigt.
Ausbeute: 4,55 g (72,1 % der Theorie),
Schmelzpunkt: 94-96 °C.

Beispiel G

2,3-Dimethoxy-7-hydroxy-7-(3-(pyranyl-2-oxy)-propin-1-yl)-6,7,8,9-tetrahydro-5H-benzocyclohepten

Hergestellt aus 2,3-Dimethoxy-6,7,8,9-tetrahydro-5H-benzocyclohepten-7-on und 3-(Pyranyl-2-oxy)-propin analog Beispiel B.
Ausbeute: 96,5 % der Theorie,
$R_F$-Wert: 0,67 (Aluminiumoxid, Laufmittel: Essigsäureethylester).

Beispiel H

2,3-Dimethoxy-7-methoxy-7-(3-(pyranyl-2-oxy)-propin-1-yl)-6,7,8,9-tetrahydro-5H-benzocyclohepten

Zu einer Lösung von 8,7 ml (13,9 mMol) n-Butyllithium in n-Hexan (ca. 15%ig) und 9,6 ml Tetrahydrofuran werden bei -30 °C 4,7 g (13 mMol) 2,3-Dimethoxy-7-hydroxy-7-(3-(pyranyl-2-oxy)-propin-1-yl)-6,7,8,9-tetrahydro-5H-benzocyclohepten, gelöst in 80 ml Tetrahydrofuran, zugetropft. 10 Minuten später tropft man 41,4 ml Dimethylsulfoxid bei -25 °C zu und weitere 8 Minuten später 1,22 ml (19,6 mMol) Methyljodid. Es wird eine Stunde bei 0-15 °C und eine Stunde bei 50 °C nachgeführt. Der Ansatz wird auf gesättigte Natriumchlorid-Lösung gegossen und mit Diethyleter extrahiert. Die organische Phase wird mit Wasser gewaschen, über Magnesiumsulfat getrocknet und im Vakuum eingedampft. Das erhaltene Rohprodukt wird über 400 g Aluminiumoxid (neutral, Aktivität II-III) mit Methylenchlorid und ansteigenden Anteilen von Ethanol (bis 3 %) gereinigt.
Ausbeute: 3,5 g (72,0 % der Theorie),
Schmelzpunkt: 67-72 °C.

Beispiel I

2,3-Dimethoxy-7-methoxy-7-(3-chlor-propin-1-yl)-6,7,8,9-tetrahydro-5H-benzocyclohepten

Eine Lösung von 3,25 g (8,68 mMol) 2,3-Dimethoxy-7-methoxy-7-(3-(pyranyl-2-oxy)-propin-1-yl)-6,7,8,9-tetrahydro-5H-benzocyclohepten und 6 ml (82 mMol) Thionylchlorid in 30 ml Chloroform wird eine Stunde unter Rückfluß erhitzt. Das im Vakuum eingedampfte Rohprodukt wird über 350 g Aluminiumoxid (neutral, Aktivität II-III) mit Methylenchlorid und 50 % Cyclohexan gereinigt.
Ausbeute: 2,0 g (74,6 % der Theorie),
Schmelzpunkt: 66-70 °C.

Beispiel J

2,3-Dimethoxy-7-(3-(pyranyl-2-oxy)-propin-1-yl)-8,9-dihydro-5H-benzocyclohepten

Hergestellt aus 2,3-Dimethoxy-7-hydroxy-7-(3-(pyranyl-2-oxy)-propin-1-yl)-6,7,8,9-tetrahydro-5H-benzocyclohepten und Methansulfonsäurechlorid in Pyridin bei 45 °C.
Ausbeute: 68,2 % der Theorie
$R_f$-Wert 0,62 (Aluminiumoxid, Laufmittel: Methylenchlorid)

Beispiel K

2,3-Dimethoxy-6,7-epoxy-7-(3-(pyranyl-2-oxy)-propin-1-yl)-6,7,8,9-tetrahydro-5H-benzocyclohepten

27 g (0,078 Mol) 2,3-Dimethoxy-7-(3-(pyranyl-2-oxy)-propin-1-yl)-8,9-dihydro-5H-benzocyclohepten, gelöst in 800 ml Chloroform, werden mit 36,6 g (0,2 Mol) m-Chlorperoxybenzoesäure, gelöst in 1000 ml Chloroform, versetzt, und 14 Stunden bei Raumtemperatur gerührt. Das restliche Peroxid wird mit 400 ml 10 %iger Natriumhydrogensulfitlösung zersetzt. Die organische Phase wird mit gesättigter Natriumhydrogencarbonat-Lösung und mit Wasser gewaschen, über Magnesiumsulfat getrocknet und im Vakuum eingedampft. Das Rohprodukt reinigt man über 1600 g Aluminiumoxid (neutral, Aktivität II-III) mit Methylenchlorid.
Ausbeute: 9,3 g (33 % der Theorie),
$R_f$-Wert 0,48 (Aluminiumoxid, Laufmittel: Methylenchlorid)

Beispiel L

2,3-Dimethoxy-7-(3-(pyranyl-2-oxy)-propin-1-yl)-6,7,8,9-tetrahydro-5H-benzocyclohepten-6-on

9,25 g (25,8 mMol) 2,3-Dimethoxy-6,7-epoxy-7-(3-(pyranyl-2-oxy)-propin-1-yl)-6,7,8,9-tetrahydro-5H-benzocyclohepten werden in 700 ml Toluol gelöst und mit 2 g (10,5 mMol) p-Toluolsulfonsäurehydrat 13 Stunden bei Raumtemperatur gerührt. Nach Extraktion mit gesättigter Natriumhydrogencarbonat-Lösung wird über Magnesiumsulfat getrocknet, im Vakuum einge dampft und das Rohprodukt über 1000 g Aluminiumoxid (neutral, Aktivität II-III) mit Cyclohexan und Methylenchlorid (50/50) gereinigt.
Ausbeute: 1,9 g (20,5% der Theorie),
$R_f$-Wert: 0,64 (Aluminiumoxid, Laufmittel: Methylenchlorid und 3 % Ethanol)

Beispiel M

2,3-Dimethoxy-7-(3-methansulfonyloxy-propin-1-yl)-6,7,8,9-tetrahydro-5H-benzocyclohepten-6-on

Hergestellt aus 2,3-Dimethoxy-7-(3-(pyranyl-2-oxy)-propin-1-yl)-6,7,8,9-tetrahydro-5H-benzocyclohepten-6-on und Methansulfonsäurechlorid in Pyridin bei 45° C.
Ausbeute: 12,7 % der Theorie.

Beispiel N

2,3-Dimethoxy-7-[3-(N-methyl-N-(2-(3,4-dimethoxy-phenyl)-ethyl)-amino)-propin-1-yl]-6,7,8,9-tetrahydro-5H-benzocyclohepten-6-on

Hergestellt aus 2,3-Dimethoxy-7-(3-methansulfonyloxy-propin-1-yl)-6,7,8,9-tetrahydro-5H-benzocyclohepten-6-on und 2 Äquivalenten N-Methyl-N-(2-(3,4-dimethoxy-phenyl)-ethyl)-amin bei 80° C.
Ausbeute: 7,0 % der Theorie.

Beispiel 1

2,3-Dimethoxy-7-[3-(N-methyl-N-(3-phenyl-propyl)-amino)-propyl]-6,7,8,9-tetrahydro-5H-benzocyclohepten-hydrochlorid

3,2 g (0,0079 Mol) 2,3-Dimethoxy-7-hydroxy-7-(3-(N-cinnamyl-N-methyl-amino)-propin-1-yl)-6,7,8,9-tetrahydro-5H-benzocyclohepten werden in 40 ml Eisessig und 2 ml Perchlorsäure in Anwesenheit von 0,5 g 10%igem Palladium auf Aktivkohle 2 Stunden bei 60° C und 3,5 bar Wasserstoff hydriert. Das im Vakuum eingedampfte Filtrat wird in Methylenchlorid gelöst und mit gesättigter Natriumhydrogencarbonat-Lösung gewaschen. Die organische Phase wird über Magnesiumsulfat getrocknet und das im Vakuum eingedampfte Rohprodukt über 280 g Aluminiumoxid (neutral, Aktivität II-III) mit Methylenchlorid gereinigt. Mit Essigester/etherischer Salzsäure wird anschließend das Hydrochlorid gefällt.
Ausbeute: 1,77 g (51,9 % der Theorie),
Schmelzpunkt: 159-160,5° C

| | | | | | | | | |
|---|---|---|---|---|---|---|---|---|
| Ber.: | C | 72,28 | H | 8,87 | N | 3,24 | Cl | 8,21 |
| Gef.: | | 72,40 | | 8,82 | | 3,19 | | 8,36 |

Beispiel 2

2,3-Dimethoxy-7-[3-(N-methyl-N-(2-(3,4-dimethoxy-phenyl)-ethyl)-amino)-propin-1-yl]-8,9-dihydro-5H-benzocyclohepten

Eine Lösung von 1,1 g (0,0025 Mol) 2,3-Dimethoxy-7-hydroxy-7-[3-(N-methyl-N-(2-(3,4-dimethoxy-phenyl)-ethyl)-amino)-propin-1-yl]-6,7,8,9-tetrahydro-5H-benzocyclohepten in 15 ml Pyridin wird bei Raumtemperatur mit 0,2 ml Methansulfonsäurechlorid versetzt und 2 Stunden bei 45° C nachgerührt. Das im Vakuum eingedampfte Rohprodukt wird in Methylenchlorid/Wasser gelöst, die organische Phase über Magnesiumsulfat ge trocknet, im Vakuum eingedampft und über 100 g Aluminiumoxid (neutral, Aktivität II-III) mit Methylenchlorid und ansteigenden Anteilen von Ethanol (bis 1 %) gereinigt.
Ausbeute: 0,66 g (60,6 % der Theorie),

| | | | | | | |
|---|---|---|---|---|---|---|
| Ber.: | C | 74,45 | H | 7,64 | N | 3,22 |
| Gef.: | | 74,30 | | 7,49 | | 3,02 |

Beispiel 3

2,3-Dimethoxy-7-hydroxy-7-[3-(N-cinnamyl-N-methyl-amino)-propyl]-6,7,8,9-tetrahydro-5H-benzocyclohepten-hydrochlorid

1,0 g (0,003 Mol) 2,3-Dimethoxy-7-hydroxy-7-[3-(N-methyl-amino)-propyl]-6,7,8,9-tetrahydro-5H-benzocyclohepten, 0,5 g (0,003 Mol) Cinnamylchlorid und 0,34 g (0,003 Mol) Triethylamin werden eine Stunde auf 80° C erhitzt. Der abgekühlte Ansatz wird mit 2 molarer Natronlauge versetzt und mit Methylenchlorid extrahiert. Die organische Phase wird über Magnesiumsulfat getrocknet und im Vakuum eingedampft. Das erhaltene Rohprodukt wird über 160 g Aluminiumoxid (neutral, Aktivität II-III) mit Methylenchlorid gereinigt, und mit etherischer Salzsäure in Aceton das Hydrochlorid gefällt.
Ausbeute: 300 mg (24 % der Theorie),
Schmelzpunkt: 234° C

| | | | | | | | | |
|---|---|---|---|---|---|---|---|---|
| Ber.: | C | 70,01 | H | 8,14 | N | 3,14 | Cl | 7,95 |
| Gef.: | | 69,86 | | 8,25 | | 3,16 | | 8,00 |

Beispiel 4

2,3-Dimethoxy-7-hydroxy-7-[3-(N-methyl-N-(3-(4-brom-phenyl)-propyl)-amino)-propyl]-6,7,8,9-tetrahydro-5H-benzocyclohepten-hydrochlorid

Hergestellt aus 3-(4-Brom-phenyl)-1-brom-propan und 2,3-Dimethoxy-7-hydroxy-7-[3-(N-methyl-amino)-propyl]-6,7,8,9-tetrahydro-5H-benzocyclohepten analog Beispiel 3.
Ausbeute: 25 % der Theorie,
Schmelzpunkt: 174° C,

| | | | | | | | | | | |
|---|---|---|---|---|---|---|---|---|---|---|
| Ber.: | C | 59,26 | H | 7,08 | N | 2,66 | Cl | 6,73 | Br | 15,17 |
| Gef.: | | 60,04 | | 7,18 | | 2,78 | | 6,43 | | 14,94 |

Beispiel 5

2,3-Dimethoxy-7-methoxy-7-[3-(N-methyl-N-(2-(3,4-dimethoxy-phenyl)-ethyl)-amino)-propin-1-yl]-6,7,8,9-tetrahydro-5H-benzocyclohepten

1,75 g (5,67 Mol) 2,3-Dimethoxy-7-methoxy-7-(3-chlor-propin-1-yl)-6,7,8,9-tetrahydro-5H-benzocyclohepten und 2,21 g (11,3 mMol) N-Methyl-2-(3,4-dimethoxy-phenyl)-ethylamin werden 1,5 Stunden auf 95 °C erhitzt. Das abgekühlte Rohprodukt wird über 400 g Aluminiumoxid (neutral, Aktivität II-III) mit Methylenchlorid und ansteigenden Anteilen von Ethanol (bis 1 %) gereinigt.
Ausbeute: 2,4 g (90,6 % der Theorie),
Schmelzpunkt: 67-70 °C,

| | | | | | | |
|---|---|---|---|---|---|---|
| Ber.: | C | 71,92 | H | 7,98 | N | 3,00 |
| Gef.: | | 71,80 | | 7,88 | | 3,00 |

Beispiel 6

2,3-Dimethoxy-7-hydroxy-7-[3-(N-methyl-N-(2-(3-trifluormethansulfonyloxy-phenyl)-ethyl)-amino)-propyl]-6,7,8,9-tetrahydro-5H-benzocyclohepten-hydrochlorid

1,0 g (0,0024 Mol) 2,3-Dimethoxy-7-hydroxy-7-[3(-N-methyl-N-(2-(3-hydroxy-phenyl)-ethyl)-amino)-propyl]-6,7,8,9-tetrahydro-5H-benzocyclohepten, 0,5 g (0,0048 Mol) Triethylamin werden in 20 ml Methylenchlorid gelöst und 0,59 g (0,0035 Mol) Trifluormethansulfonsäurechlorid zugetropft. Nach 16 Stunden wird die Lösung im Vakuum eingedampft, das Rohprodukt über 160 g Aluminiumoxid (neutral, Aktivität II-III) mit Methylenchlorid gereinigt, und das Hydrochlorid mit etherischer Salzsäure in Aceton gelöst.
Ausbeute: 490 mg (97 % der Theorie),
Schmelzpunkt: 160 °C,

| | | | | | | | | | | |
|---|---|---|---|---|---|---|---|---|---|---|
| Ber.: | C | 53,65 | H | 6,06 | N | 2,41 | Cl | 6,09 | S | 5,51 |
| Gef.: | | 53,53 | | 6,08 | | 2,55 | | 6,28 | | 5,80 |

Beispiel 7

a) N-(3-(2,3-Dimethoxy-7-hydroxy-6,7,8,9-tetrahydro-5H-benzocyclohepten-7-yl)-propyl)-N-methyl-3,4-dimethoxy-zimtsäureamid

1,2 g (0,006 Mol) 3,4-Dimethoxy-zimtsäure werden in 60 ml Essigester suspendiert, mit 1,0 g (0,006 Mol) N,N′-Carbonyldiimidazol versetzt und 15 Minuten bei 40 °C gerührt. Zu dieser Suspension werden 2,0 g (0,006 Mol) 2,3-Dimethoxy-7-hydroxy-7-[3-(N-methyl-amino)-propyl]-6,7,8,9-tetrahydro-5H-benzocyclohepten getropft und 28 Stunden unter Rückfluß gekocht. Den abgekühlten Ansatz versetzt man mit 2 molarer Na tronlauge und extrahiert mehrmals mit Essigester. Die organischen Phasen trocknet man über Magnesiumsulfat und dampft im Vakuum ein. Das Rohprodukt wird über 380 g Aluminiumoxid (neutral, Aktivität II-III) mit Methylenchlorid gereinigt.
Ausbeute: 1.3 g (45 % der Theorie),
Schmelzpunkt: 145-146 °C,
$R_F$-Wert: 0,6 (Aluminiumoxid, Laufmittel: 5 % Ethanol in Methylenchlorid).

b) 2,3-Dimethoxy-7-hydroxy-7-[3-(N-methyl-N-(3,4-dimethoxy-cinnamyl)-amino)-propyl]-6,7,8,9-tetrahydro-5H-benzocyclohepten-hydrochlorid

Zu 0,11 g (0,003 Mol) Lithiumaluminiumhydrid in 15 ml trockenem Tetrahydrofuran werden bei 5 °C 1,3 g (0,0027 Mol) N-(3-(2,3-Dimethoxy-7-hydroxy-6,7,8,9-tetrahydro-5H-benzocyclohepten-7-yl)-propyl)-N-methyl-3,4-dimethoxy-zimtsäureamid, gelöst in 10 ml trockenem Tetrahydrofuran, getropft. Nach 19 Stunden Rühren bei Raumtemperatur werden unter Eiskühlung nacheinander 0,1 ml Wasser, 0,1 ml 15%ige Natronlauge und 0,3 ml Wasser zugetropft. Der Niederschlag wird abgesaugt, mit Tetrahydrofuran gewaschen und das Filtrat im Vakuum eingedampft. Das Rohprodukt wird über 160 g Aluminiumoxid (neutral, Aktivität II-III) mit Methylenchlorid gereinigt, dann mit etherischer Salzsäure in Aceton das Hydrochlorid gefällt.
Ausbeute: 80 mg (6 % der Theorie),
Schmelzpunkt: 199 °C,

| | | | | | | | | |
|---|---|---|---|---|---|---|---|---|
| Ber.: | C | 66,45 | H | 7,97 | N | 2,77 | Cl | 7,01 |
| Gef.: | | 66,30 | | 8,81 | | 2,89 | | 7,05 |

Beispiel 8

2,3-Dimethoxy-7-hydroxy-7-[3-(N-methyl-N-(2-(3,4-dimethoxy-phenyl)-ethyl)-amino)-propin-1-yl]-6,7,8,9-tetrahydro-5H-benzocyclohepten

Zu einer aus 0,26 g (10,6 mMol) Magnesium in 2 ml Diethylether und 0,85 ml (10,6 mMol) Ethylbromid in 1 ml Diethylether frisch hergestellten Lösung von Ethylmagnesiumbromid werden bei 15 °C 3,8 ml Tetrahydrofuran zugetropft. Anschliessend wird bei 20-27 °C eine Lösung von 2,47 g (10,6 mMol) 3-(N-Methyl-N-(2-(3,4-dimethoxy-phenyl)-ethyl)-amino)-propin in 2 ml Tetrahydrofuran zugetropft. Nach beeendeter Ethanentwicklung tropft man 1,87 g (8,5 mMol) 2,3-Dimethoxy-6,7,8,9-tetrahydro-5H-benzocyclohepten-7-on in 4 ml Tetrahydrofuran zu. Nach 30 Minuten bei ca. 30 °C wird mit 15 ml 10%iger Ammoniumchlorid-Lösung versetzt, mit Diethylether extrahiert, mit halb gesättigter Kaliumcarbonat-Lösung und mit Wasser gewaschen. Die organische Phase wird über Magnesiumsulfat getrocknet und im Vakuum eingedampft. Das erhaltene Rohprodukt wird über 400 g Aluminiumoxid (neutral, Aktivität II-III) mit Methylenchlorid und anschließend ansteigenden Anteilen von Ethanol (bis 3 %) gereinigt.
Ausbeute: 2,27 g (59,0 % der Theorie),
Schmelzpunkt: 114-117 °C,

| | | | | | | |
|---|---|---|---|---|---|---|
| Ber.: | C | 71,50 | H | 7,78 | N | 3,09 |
| Gef.: | | 71,40 | | 7,55 | | 2,86 |

Beispiel 9

2,3-Dimethoxy-7-hydroxy-7-[(3-(N-cinnamyl-N-methyl)-amino)-propin-1-yl]-6,7,8,9-tetrahydro-5H-benzocyclohepten-hydrochlorid

Hergestellt aus 3-[(N-Cinnamyl-N-methyl-amino)-prop-1-in und 2,3-Dimethoxy-6,7,8,9-tetrahydro-5H-benzocyclohepten-7-on analog Beispiel 8.
Ausbeute: 39 % der Theorie,
Schmelzpunkt: 214 °C,

| | | | | | | | | |
|---|---|---|---|---|---|---|---|---|
| Ber.: | C | 70,65 | H | 7,29 | N | 3,16 | Cl | 8,02 |
| Gef.: | | 70,53 | | 7,43 | | 2,98 | | 8,28 |

Beispiel 10

2,3-Dimethoxy-7-hydroxy-7-[3-(N-methyl-N-(2-(3,5-dimethoxy-phenyl)-ethyl)-amino)-propin-1-yl]-6,7,8,9-tetrahydro-5H-benzocyclohepten

Hergestellt aus 2,3-Dimethoxy-6,7,8,9-tetrahydro-5H-benzocyclohepten-7-on und 3-(N-Methyl-N-(2-(3,5-dimethoxy-phenyl)-ethyl)-amino)-propin analog Beispiel 8.
Ausbeute: 42,7 % der Theorie,
Schmelzpunkt: 112-113° C,

| | | | | | | |
|---|---|---|---|---|---|---|
| Ber.: | C | 71,50 | H | 7,78 | N | 3,09 |
| Gef.: | | 71,30 | | 7,80 | | 2,88 |

Beispiel 11

2,3-Dimethoxy-7-hydroxy-7-[3-(N-methyl-N-(2-(4-benzyloxy-phenyl)-ethyl)-amino)-propin-1-yl]-6,7,8,9-tetrahydro-5H-benzocyclohepten

Hergestellt aus 2,3-Dimethoxy-6,7,8,9-tetrahydro-5H-benzocyclohepten-7-on und 3-(N-Methyl-N-(2-(4-benzyloxy-phenyl)-ethyl)-amino)-propin analog Beispiel 8.
Ausbeute: 81,8 % der Theorie,
Schmelzpunkt: 86-88° C,

| | | | | | | |
|---|---|---|---|---|---|---|
| Ber.: | C | 76,92 | H | 7,46 | N | 2,80 |
| Gef.: | | 76,76 | | 7,46 | | 2,81 |

Beispiel 12

2,3-Dimethoxy-7-hydroxy-7-[3-(N-methyl-N-(2-(4-methoxy-phenyl)-ethyl)-amino)-propin-1-yl]-6,7,8,9-tetrahydro-5H-benzocyclohepten

Hergestellt aus 2,3-Dimethoxy-6,7,8,9-tetrahydro-5H-benzocyclohepten-7-on und 3-(N-Methyl-N-(2-(4-methoxy-phenyl)-ethyl)-amino)-propin analog Beispiel 8.
Ausbeute: 64,4 % der Theorie,
Schmelzpunkt: 101-104° C,

| | | | | | | |
|---|---|---|---|---|---|---|
| Ber.: | C | 73,73 | H | 7,85 | N | 3,31 |
| Gef.: | | 73,69 | | 8,02 | | 3,55 |

Beispiel 13

2,3-Dimethoxy-7-hydroxy-7-[3-(N-methyl-N-(2-(4-methoxy-phenyl)-ethyl)-amino)-propyl]-6,7,8,9-tetrahydro-5H-benzocyclohepten-oxalat

3,3 g (0,008 ml) 2,3-Dimethoxy-7-hydroxy-7-[3-(N-methyl-N-(2-(4-methoxy-phenyl)-ethyl)-amino)-propin-1-yl]-6,7,8,9-tetrahydro-5H-benzocyclohepten werden in 40 ml Eisessig in Anwesenheit von 0,3 g 10%igem Palladium auf Aktivkohle 0,5 Stunden bei Raumtemperatur und 1 bar Wasserstoff hydriert. Das im Vakuum eingedampfte Filtrat wird in Methylenchlorid gelöst und mit gesättigter Natriumhydrogencarbonatlösung

extrahiert. Die organische Phase wird über Magnesiumsulfat getrocknet, im Vakuum eingedampft und das erhaltene Rohprodukt über 300 g Aluminiumoxid (neutral, Aktivität II-III) mit Methylenchlorid und ansteigenden Anteilen von Ethanol (bis 15 %) gereinigt. Anschließend wird in Essigester/Diethylether mit Oxalsäure das Oxalat gefällt.
Ausbeute: 1,64 g (73,9 % der Theorie),
Schmelzpunkt: > 40 °C (Sinterung),

| | | | | | | |
|---|---|---|---|---|---|---|
| Ber.: | C | 64,97 | H | 7,59 | N | 2,71 |
| Gef.: | | 65,00 | | 7,72 | | 2,52 |

Beispiel 14

2,3-Dimethoxy-7-hydroxy-7-[3-(N-methyl-N-(2-(4-hydroxy-phenyl)-ethyl)-amino)-propyl]-6,7,8,9-tetrahydro-5H-benzocyclohepten-oxalat

Hergestellt aus 2,3-Dimethoxy-7-hydroxy-7-[3-(N-methyl-N-(2-(4-benzyloxy-phenyl)-ethyl)-amino)-propin-1-yl]-6,7,8,9-tetrahydro-5H-benzocyclohepten analog Beispiel 13.
Ausbeute: 81,3 % der Theorie,

| | | | | | | |
|---|---|---|---|---|---|---|
| Ber.: | C | 72,61 | H | 8,53 | N | 3,99 |
| Gef.: | | 72,49 | | 8,79 | | 3,74 |

$R_F$-Wert: 0,28 (Aluminiumoxid, Laufmittel: 3 % Ethanol in Methylenchlorid)

Beispiel 15

2,3-Dimethoxy-7-[3-(N-methyl-N-(2-(4-methoxy-phenyl)-ethyl)-amino)-propyl]-6,7,8,9-tetrahydro-5H-benzocyclohepten-hydrochlorid

Hergestellt aus dem Isomerengemisch von 2,3-Dimethoxy-7-[3-(N-methyl-N-(2-(4-methoxy-phenyl)-ethyl)-amino)-propyl]-8,9-dihydro-5H-benzocyclohepten und 2,3-Dimethoxy-7-[3-(N-methyl-N-(2-(4-methoxy-phenyl)-ethyl)-amino)-propyliden]-5,6,8,9-tetrahydro-benzocyclohepten analog Beispiel 16.
Ausbeute: 55,6 % der Theorie,
Schmelzpunkt: 158-160 °C,

| | | | | | | | | |
|---|---|---|---|---|---|---|---|---|
| Ber.: | C | 69,70 | H | 8,55 | N | 3,13 | Cl | 7,91 |
| Gef.: | | 68,95 | | 8,60 | | 3,17 | | 7,40 |

Beispiel 16

2,3-Dimethoxy-7-[3-(N-methyl-N-(2-(3,4-dimethoxy-phenyl)-ethyl)-amino)-propyl]-6,7,8,9-tetrahydro-5H-benzocyclohepten-hydrochlorid

1,65 mg (3,75 mMol) eines Isomerengemisches aus 2,3-Dimethoxy-7-[3-(N-methyl-N-(2-(3,4-dimethoxy-phenyl)-ethyl)-amino)-propyl]-8,9-dihydro-5H-benzocyclohepten und 2,3-Dimethoxy-7-[3-(N-methyl-N-(2-(3,4-dimethoxy-phenyl)-ethyl)-amino)-propyliden]-5,6,8,9-tetrahydro-benzocyclohepten werden in 20 ml Ethanol in Anwesenheit von 0,2 g 10%igem Palladium auf Aktivkohle 7 Stunden bei Raumtemperatur und 5 bar

Wasserstoff hydriert. Das Filtrat wird im Vakuum eingedampft und mit Aceton/etherischer Salzsäure das Hydrochlorid gefällt. Ausbeute: 0,85 g (47,5 % der Theorie),
Schmelzpunkt: 155-156 °C,

| | | | | | | | | |
|---|---|---|---|---|---|---|---|---|
| Ber.:<br>Gef.: | C | 67,83<br>67,85 | H | 8,43<br>8,33 | N | 2,93<br>2,97 | Cl | 7,42<br>7,76 |

Beispiel 17

2,3-Dimethoxy-7-hydroxy-7-[3-(N-methyl-N-(2-(3,4-dimethoxy-phenyl)-ethyl)-amino)-propyl]-6,7,8,9-tetrahydro-5H-benzocyclohepten

Hergestellt aus 2,3-Dimethoxy-7-hydroxy-7-[3-(N-methyl-N-(2-(3,4-dimethoxy-phenyl)-ethyl)-amino)-propin-1-yl]-6,7,8,9-tetrahydro-5H-benzocyclohepten analog Beispiel 13.
Ausbeute: 60,6 % der Theorie,
Schmelzpunkt: 91 °C,

| | | | | | | |
|---|---|---|---|---|---|---|
| Ber.:<br>Gef.: | C | 70,87<br>70,87 | H | 8,59<br>8,46 | N | 3,06<br>2,92 |

Beispiel 18

2,3-Dimethoxy-7-[3-(N-methyl-N-(2-(3-methoxy-phenyl)-ethyl)-amino)-propyl]-6,7,8,9-tetrahydro-5H-benzocyclohepten-hydrochlorid

Hergestellt aus dem Isomerengemisch von 2,3-Dimethoxy-7-[3-(N-methyl-N-(2-(3-methoxy-phenyl)-ethyl)-amino)-propyl]-8,9-dihydro-5H-benzocyclohepten und 2,3-Dimethoxy-7-[3-(N-methyl-N-(2-(3-methoxy-phenyl)-ethyl)-amino)-propyliden]-5,6,8,9-tetrahydro-benzocyclohepten analog Beispiel 16.
Ausbeute: 76,9 % der Theorie,
Schmelzpunkt: 164-165 °C,

| | | | | | | | | |
|---|---|---|---|---|---|---|---|---|
| Ber.:<br>Gef.: | C | 69,70<br>69,63 | H | 8,55<br>8,53 | N | 3,13<br>3,11 | Cl | 7,91<br>8,02 |

Beispiel 19

2,3-Dimethoxy-7-[3-(N-methyl-N-(2-(4-hydroxy-phenyl)-ethyl)-amino)-propyl]-6,7,8,9-tetrahydro-5H-benzocyclohepten-hydrochlorid

Hergestellt aus dem Isomerengemisch von 2,3-Dimethoxy-7-[3-(N-methyl-N-(2-(4-hydroxy-phenyl)-ethyl)-amino)-propyl]-8,9-dihydro-5H-benzocyclohepten und 2,3-Dimethoxy-7-[3-(N-methyl-N-(2-(4-hydroxy-phenyl)-ethyl)-amino)-propyliden]-5,6,8,9-tetrahydro-benzocyclohepten analog Beispiel 16.
Ausbeute: 55,8 % der Theorie,
Schmelzpunkt: 204-206 °C,

| | | | | | | | | |
|---|---|---|---|---|---|---|---|---|
| Ber.: | C | 69,18 | H | 8,36 | N | 3,23 | Cl | 8,17 |
| Gef.: | | 68,98 | | 8,57 | | 3,03 | | 8,28 |

Beispiel 20

2,3,7-Trimethoxy-7-[3-(N-methyl-N-(2-(3,4-dimethoxy-phenyl)-ethyl)-amino)-propyl]-6,7,8,9-tetrahydro-5H-benzocyclohepten-hydrochlorid

Hergestellt aus 2,3,7-Trimethoxy-7-[3-(N-methyl-N-(2-(3,4-dimethoxy-phenyl)-ethyl)-amino)-propin-1-yl]-6,7,8,9-tetrahydro-5H-benzocyclohepten-hydrochlorid analog Beispiel 13.
Ausbeute: 83,1 % der Theorie,
Schmelzpunkt: 135-137° C,

| | | | | | | | | |
|---|---|---|---|---|---|---|---|---|
| Ber.: | C | 66,18 | H | 8,33 | N | 2,76 | Cl | 6,98 |
| Gef.: | | 66,36 | | 8,18 | | 2,81 | | 6,92 |

Beispiel 21

2,3-Dimethoxy-7-[3-(N-methyl-N-(2-(4-fluor-phenyl)-ethyl)-amino)-propyl]-6,7,8,9-tetrahydro-5H-benzocyclohepten-hydrochlorid

Hergestellt aus 2,3-Dimethoxy-7-(3-(N-methyl-amino)-propyl)-6,7,8,9-tetrahydro-5H-benzocyclohepten und Methansulfonsäure-2-(4-fluor-phenyl)-ethylester analog Beispiel 3.
Ausbeute: 30,3 % der Theorie,
Schmelzpunkt: 122-124° C,

| | | | | | | | | |
|---|---|---|---|---|---|---|---|---|
| Ber.: | C | 68,87 | H | 8,09 | N | 3,21 | Cl | 8,13 |
| Gef.: | | 68,75 | | 8,18 | | 3,12 | | 8,15 |

Beispiel 22

2,3-Dimethoxy-7-hydroxy-7-[3-(N-methyl-N-(2-(4-benzyloxy-phenyl)-ethyl)-amino)-propyl]-6,7,8,9-tetrahydro-5H-benzocyclohepten-hydrochlorid

Hergestellt aus 2,3-Dimethoxy-7-hydroxy-7-(3-(N-methyl-amino)-propyl)-6,7,8,9-tetrahydro-5H-benzocyclohepten und Methansulfonsäure-2-(4-benzyloxy-phenyl)-ethylester analog Beispiel 3.
Ausbeute: 30,1 % der Theorie,
Schmelzpunkt: 170-171° C,

| | | | | | | | | |
|---|---|---|---|---|---|---|---|---|
| Ber.: | C | 71,18 | H | 7,84 | N | 2,59 | Cl | 6,57 |
| Gef.: | | 70,93 | | 7,91 | | 2,57 | | 6,67 |

Beispiel 23

2,3-Dimethoxy-7-hydroxy-7-[3-(N-methyl-N-(2-(4-amino-3,5-dichlor-phenyl)-ethyl)-amino)-propyl]-6,7,8,9-tetrahydro-5H-benzocyclohepten-oxalat

Hergestellt aus 2,3-Dimethoxy-7-hydroxy-7-(3-(N-methyl-amino)-propyl)-6,7,8,9-tetrahydro-5H-benzocyclohepten und 2-(4-Amino-3,5-dichlor-phenyl)-ethylbromid analog Beispiel 3.
Ausbeute: 36,8 % der Theorie,
Schmelzpunkt: >75 °C (Zers.),

| | | | | | | | | |
|---|---|---|---|---|---|---|---|---|
| Ber.: | C | 56,74 | H | 6,35 | N | 4,90 | Cl | 12,41 |
| Gef.: | | 56,57 | | 6,58 | | 4,74 | | 12,59 |

Beispiel 24

2,3-Dimethoxy-7-hydroxy-7-[3-(N-methyl-N-(3-(4-amino-3,5-dibrom-phenoxy)-propyl)-amino)-propyl]-6,7,8,9-tetrahydro-5H-benzocyclohepten-oxalat

Hergestellt aus 2,3-Dimethoxy-7-hydroxy-7-(3-(N-methyl-amino)-propyl)-6,7,8,9-tetrahydro-5H-benzocyclohepten und 3-(4-Amino-3,5-dibrom-phenoxy)-propylchlorid analog Beispiel 3.
Ausbeute: 44,9 % der Theorie,
Schmelzpunkt: >80 °C (Zers.),

| | | | | | | | | |
|---|---|---|---|---|---|---|---|---|
| Ber.: | C | 48,71 | H | 5,55 | N | 4,06 | Br | 23,15 |
| Gef.: | | 48,59 | | 5,70 | | 3,91 | | 23,42 |

Beispiel 25

2,3-Dimethoxy-7-hydroxy-7-[3-(N-methyl-N-(3-(4-cyano-phenyl)-propyl)-amino)-propyl]-6,7,8,9-tetrahydro-5H-benzocyclohepten

Hergestellt aus 2,3-Dimethoxy-7-hydroxy-7-(3-(N-methyl-amino)-propyl)-6,7,8,9-tetrahydro-5H-benzocyclohepten und 3-(4-Cyano-phenyl)-propylbromid analog Beispiel 3.
Ausbeute: 86,2 % der Theorie,
Schmelzpunkt: 79-81 °C,

| | | | | | | |
|---|---|---|---|---|---|---|
| Ber.: | C | 74,28 | H | 8,31 | N | 6,42 |
| Gef.: | | 74,23 | | 8,19 | | 6,52 |

Beispiel 26

2,3-Dimethoxy-7-hydroxy-7-[3-(N-methyl-N-(2-(4-nitro-phenyl)-ethyl)-amino)-propyl]-6,7,8,9-tetrahydro-5H-benzocyclohepten

Hergestellt aus 2,3-Dimethoxy-7-hydroxy-7-(3-(N-methyl-amino)-propyl)-6,7,8,9-tetrahydro-5H-benzocyclohepten und 2-(4-Nitro-phenyl)-ethylbromid analog Beispiel 3.
Ausbeute: 46,3 % der Theorie,

| | | | | | | |
|---|---|---|---|---|---|---|
| Ber.: | C | 67,85 | H | 7,74 | N | 6,33 |
| Gef.: | | 67,75 | | 7,98 | | 6,55 |

$R_F$-Wert: 0,25 (Aluminiumoxid, Laufmittel: Cyclohexan + 50 % Essigester)

Beispiel 27

2,3-Dimethoxy-7-[3-(N-methyl-N-(3-(4-amino-3,5-dibrom-phenoxy)-propyl)-amino)-propyl]-6,7,8,9-tetrahydro-5H-benzocyclohepten-hydrochlorid

Hergestellt aus 2,3-Dimethoxy-7-(3-(N-methyl-amino)-propyl)-6,7,8,9-tetrahydro-5H-benzocyclohepten und 3-(4-Amino-3,5-dibrom-phenoxy)-1-chlorpropan analog Beispiel 3.
Ausbeute: 21,8 % der Theorie,
Schmelzpunkt: 80 °C (Zers.),

| | | | | | | | | | | |
|---|---|---|---|---|---|---|---|---|---|---|
| Ber.: | C | 50,30 | H | 6,01 | N | 4,51 | Cl | 5,71 | Br | 25,74 |
| Gef.: | | 50,21 | | 6,00 | | 4,49 | | 5,65 | | 25,48 |

Beispiel 28

2,3-Dimethoxy-7-[3-(N-methyl-N-(3-(3,4-methylendioxy-phenoxy)-propyl)-amino)-propyl]-6,7,8,9-tetrahydro-5H-benzocyclohepten-hydrochlorid

Hergestellt aus 2,3-Dimethoxy-7-(3-(N-methyl-amino)-propyl)-6,7,8,9-tetrahydro-5H-benzocyclohepten und 3-(3,4-Methylendioxy-phenoxy)-1-chlorpropan analog Beispiel 3.
Ausbeute: 21,1 % der Theorie,
Schmelzpunkt: 146-147 °C,

| | | | | | | | | |
|---|---|---|---|---|---|---|---|---|
| Ber.: | C | 65,90 | H | 7,78 | N | 2,85 | Cl | 7,21 |
| Gef.: | | 65,90 | | 7,91 | | 2,70 | | 7,39 |

Beispiel 29

2,3-Dimethoxy-7-[3-(N-methyl-N-(3-(3-methyl-phenoxy)-propyl)-amino)-propyl]-6,7,8,9-tetrahydro-5H-benzocyclohepten-hydrochlorid

Hergestellt aus 2,3-Dimethoxy-7-(3-(N-methyl-amino)-propyl)-6,7,8,9-tetrahydro-5H-benzocyclohepten und 3-(3-Methyl-phenoxy)-1-chlorpropan analog Beispiel 3.
Ausbeute: 9,7 % der Theorie,
Schmelzpunkt: 126-127 °C,

| | | | | | | | | |
|---|---|---|---|---|---|---|---|---|
| Ber.: | C | 70,18 | H | 8,73 | N | 3,03 | Cl | 7,67 |
| Gef.: | | 69,97 | | 8,81 | | 2,93 | | 7,54 |

Beispiel 30

2,3-Dimethoxy-7-[3-(N-methyl-N-(2-(4-trifluormethansulfoxy-phenyl)-ethyl)-amino)-propyl]-6,7,8,9-tetrahydro-5H-benzocyclohepten-hydrochlorid

Hergestellt aus 2,3-Dimethoxy-7-[3-(N-methyl-N-(2-(4-hydroxy-phenyl)-ethyl)-amino)-propyl]-6,7,8,9-tetrahydro-5H-benzocyclohepten und Trifluormethansulfonsäurechlorid analog Beispiel 6.
Ausbeute: 76,4 % der Theorie,
Schmelzpunkt: 144-146 °C,

| | | | | | | | | |
|---|---|---|---|---|---|---|---|---|
| Ber.: | C | 55,16 | H | 6,23 | N | 2,47 | Cl | 6,26 |
| Gef.: | | 55,16 | | 6,39 | | 2,32 | | 6,15 |

Beispiel 31

2,3-Dimethoxy-7-[3-(N-methyl-N-(2-(4-methansulfonyloxy-phenyl)-ethyl)-amino)-propyl]-6,7,8,9-tetrahydro-5H-benzocyclohepten-hydrochlorid

Hergestellt aus 2,3-Dimethoxy-7-[3-(N-methyl-N-(2-(4-hydroxy-phenyl)-ethyl)-amino)-propyl]-6,7,8,9-tetrahydro-5H-benzocyclohepten und Methansulfonsäurechlorid analog Beispiel 6.
Ausbeute: 73,2 % der Theorie,
Schmelzpunkt: 162-164 °C,

| | | | | | | | | |
|---|---|---|---|---|---|---|---|---|
| Ber.: | C | 60,98 | H | 7,48 | N | 2,74 | Cl | 6,92 |
| Gef.: | | 60,83 | | 7,47 | | 2,63 | | 6,96 |

Beispiel 32

2,3-Dimethoxy-7-[3-(N-methyl-N-(2-(4-hydroxy-3-methoxy-phenyl)-ethyl)-amino)-propyl]-6,7,8,9-tetrahydro-5H-benzocyclohepten

Hergestellt aus 2,3-Dimethoxy-7-hydroxy-7-[3-(N-methyl-N-(2-(4-benzyloxy-3-methoxy-phenyl)-ethyl)-amino)-propin-1-yl]-6,7,8,9-tetrahydro-5H-benzocyclohepten analog Beispiel 1.
Ausbeute: 67,2 % der Theorie,
Schmelzpunkt: 47 °C,

| | | | | | | |
|---|---|---|---|---|---|---|
| Ber.: | C | 73,04 | H | 8,72 | N | 3,28 |
| Gef.: | | 73,10 | | 8,68 | | 3,51 |

Beispiel 33

2,3-Dimethoxy-7-hydroxy-7-[3-(N-methyl-N-(3-(3,4-methylendioxy-phenoxy)-propyl)-amino)-propin-1-yl]-6,7,8,9-tetrahydro-5H-benzocyclohepten-hydrochlorid

Hergestellt aus 3-(N-Methyl-N-(3-(3,4-methylendioxy-phenoxy-propyl)-amino)-propin und 2,3-Dimethoxy-6,7,8,9-tetrahydro-5H-benzocyclohepten-7-on analog Beispiel 8.
Ausbeute: 30 % der Theorie,

| | | | | | | | | |
|---|---|---|---|---|---|---|---|---|
| Ber.: | C | 64,34 | H | 6,80 | N | 2,78 | Cl | 7,03 |
| Gef.: | | 64,25 | | 6,79 | | 2,57 | | 7,04 |

Beispiel 34

2,3-Dimethoxy-7-hydroxy-7-[3-(N-methyl-N-(3-(3,4-methylendioxy-phenoxy)-propyl)-amino-propyl]-6,7,8,9-tetrahydro-5H-benzocyclohepten-hydrochlorid

Hergestellt aus 2,3-Dimethoxy-7-hydroxy-7-[3-(N-methyl-N-(3-(3,4-methylendioxy-phenoxy)-propyl)-amino-propin-1-yl)-6,7,8,9-tetrahydro-5H-benzocyclohepten analog Beispiel 13.
Ausbeute: 18 % der Theorie,
Schmelzpunkt: 167 °C,

| | | | | | | | | |
|---|---|---|---|---|---|---|---|---|
| Ber.: | C | 63,83 | H | 7,54 | N | 2,76 | Cl | 6,98 |
| Gef.: | | 63,72 | | 7,66 | | 2,79 | | 7,02 |

Beispiel 35

2,3-Dimethoxy-7-hydroxy-7-[3-(N-methyl-N-(2-(4-fluor-phenyl)-ethyl)-amino)-propin-1-yl]-6,7,8,9-tetrahydro-5H-benzocyclohepten-hydrochlorid

Hergestellt aus 3-(N-Methyl-N-(2-(4-fluor-phenyl)-ethyl)-amino)-propin und 2,3-Dimethoxy-6,7,8,9-tetrahydro-5H-benzocyclohepten-7-on analog Beispiel 8.
Ausbeute: 42 % der Theorie,
Schmelzpunkt: 231 °C,

| | | | | | | | | |
|---|---|---|---|---|---|---|---|---|
| Ber.: | C | 67,03 | H | 6,98 | N | 3,13 | Cl | 7,91 |
| Gef.: | | 66,88 | | 7,17 | | 2,98 | | 8,17 |

Beispiel 36

2,3-Dimethoxy-7-hydroxy-7-[3-(N-methyl-N-(2-(4-fluor-phenyl)-ethyl)-amino)-propyl]-6,7,8,9-tetrahydro-5H-benzocyclohepten-hydrochlorid

Hergestellt aus 2,3-Dimethoxy-7-hydroxy-[3-(N-methyl-N-(2-(4-fluor-phenyl)-ethyl)-amino)-propin-1-yl]-

6,7,8,9-tetrahydro-5H-benzocyclohepten analog Beispiel 13.
Ausbeute: 28 % der Theorie,
Schmelzpunkt: 178° C,

| | | | | | | | | |
|---|---|---|---|---|---|---|---|---|
| Ber.: | C | 66,43 | H | 7,58 | N | 3,10 | Cl | 7,84 |
| Gef.: | | 66,30 | | 7,74 | | 2,94 | | 8,01 |

Beispiel 37

2,3-Dimethoxy-7-hydroxy-7-[3-(N-methyl-N-(2-(3-methoxy-phenyl)-ethyl)-amino)-propin-1-yl]-6,7,8,9-tetrahydro-5H-benzocyclohepten-hydrochlorid

Hergestellt aus 3-(N-methyl-N-(2-(3-methoxy-phenyl)-ethyl)-amino)-propin und 2,3-Dimethoxy-6,7,8,9-tetrahydro-5H-benzocyclohepten-7-on analog Beispiel 8.
Ausbeute: 40 % der Theorie,
Schmelzpunkt: 187° C,

| | | | | | | | | |
|---|---|---|---|---|---|---|---|---|
| Ber.: | C | 67,89 | H | 7,45 | N | 3,04 | Cl | 7,71 |
| Gef.: | | 67,92 | | 7,61 | | 3,03 | | 7,97 |

Beispiel 38

2,3-Dimethoxy-7-hydroxy-7-[3-(N-methyl-N-(2-(3-methoxy-phenyl)-ethyl)-amino)-propyl]-6,7,8,9-tetrahydro-5H-benzocyclohepten-hydrochlorid

Hergestellt aus 2,3-Dimethoxy-7-hydroxy-7-[3-(N-methyl-N-(2-(3-methoxy-phenyl)-ethyl)-amino)-propin-1-yl]-6,7,8,9-tetrahydro-5H-benzocyclohepten analog Beispiel 13.
Ausbeute: 65 % der Theorie,
Schmelzpunkt: 149-150° C,

| | | | | | | | | |
|---|---|---|---|---|---|---|---|---|
| Ber.: | C | 67,29 | H | 8,25 | N | 3,02 | Cl | 7,64 |
| Gef.: | | 67,44 | | 8,39 | | 3,04 | | 7,76 |

Beispiel 39

2,3-Dimethoxy-7-hydroxy-7-[3-(N-methyl-N-(3-(3,4-dimethyl-phenoxy)-propyl)-amino)-propin-1-yl]-6,7,8,9-tetrahydro-5H-benzocyclohepten

Hergestellt aus 3-(N-Methyl-N-(3-(3,4-dimethyl-phenoxy)-propyl)-amino)-propin und 2,3-Dimethoxy-6,7,8,9-tetrahydro-5H-benzocyclohepten-7-on analog Beispiel 8.
Ausbeute: 58 % der Theorie,
Schmelzpunkt: 99-100° C,

| | | | | | | |
|---|---|---|---|---|---|---|
| Ber.: | C | 74,47 | H | 8,26 | N | 3,10 |
| Gef.: | | 74,70 | | 8,23 | | 3,10 |

Beispiel 40

2,3-Dimethoxy-7-hydroxy-7-[3-(N-methyl-N-(3-(3,4-dimethyl-phenoxy)-propyl)-amino)-propyl]-6,7,8,9-tetrahydro-5H-benzocyclohepten-hydrochlorid

Hergestellt aus 2,3-Dimethoxy-7-hydroxy-7-[3-(N-methyl-N-(3-(3,4-dimethyl-phenoxy)-propyl)-amino)-propin-1-yl]-6,7,8,9-tetrahydro-5H-benzocyclohepten analog Beispiel 13.
Ausbeute: 63 % der Theorie,
Schmelzpunkt: 179° C,

| | | | | | | | | |
|---|---|---|---|---|---|---|---|---|
| Ber.: | C | 68,34 | H | 8,60 | N | 2,85 | Cl | 7,21 |
| Gef.: | | 68,21 | | 8,70 | | 2,85 | | 7,42 |

Beispiel 41

2,3-Dimethoxy-7-hydroxy-7-[3-(N-methyl-N-(3-(3-methoxy-phenoxy)-propyl)-amino)-propin-1-yl]-6,7,8,9-tetrahydro-5H-benzocyclohepten-hydrochlorid

Hergestellt aus 3-(N-Methyl-N-(3-(3-methoxy-phenoxy)-propyl)-amino)-propin und 2,3-Dimethoxy-6,7,8,9-tetrahydro-5H-benzocyclohepten-7-on analog Beispiel 8.
Ausbeute: 28 % der Theorie,
Schmelzpunkt: 168° C,

| | | | | | | | | |
|---|---|---|---|---|---|---|---|---|
| Ber.: | C | 66,18 | H | 2,86 | N | 7,40 | Cl | 7,24 |
| Gef.: | | 66,01 | | 2,76 | | 7,37 | | 7,31 |

Beispiel 42

2,3-Dimethoxy-7-hydroxy-7-[3-(N-methyl-N-(3-(3-methoxy-phenoxy)-propyl)-amino)-propyl]-6,7,8,9-tetrahydro-5H-benzocyclohepten-hydrochlorid

Hergestellt aus 2,3-Dimethoxy-7-hydroxy-7-[3-(N-methyl-N-(3-3-methoxy-phenoxy)-propyl)-amino)-propin-1-yl]-6,7,8,9-tetrahydro-5H-benzocyclohepten-hydrochlorid analog Beispiel 13.
Ausbeute: 41 % der Theorie,
Schmelzpunkt: 168° C,

| | | | | | | | | |
|---|---|---|---|---|---|---|---|---|
| Ber.: | C | 65,64 | H | 8,16 | N | 2,83 | Cl | 7,18 |
| Gef.: | | 65,55 | | 8,02 | | 2,69 | | 7,25 |

Beispiel 43

2,3-Dimethoxy-7-hydroxy-7-[3-(N-methyl-N-(2-(3-benzyloxy-phenyl)-ethyl)-amino)-propin-1-yl]-6,7,8,9-tetrahydro-5H-benzocyclohepten-hydrochlorid

Hergestellt aus 3-(N-Methyl-N-(2-(3-benzyloxy-phenyl)-ethyl)-amino)-propin und 2,3-Dimethoxy-6,7,8,9-tetrahydro-5H-benzocyclohepten-7-on analog Beispiel 8.
Ausbeute: 36 % der Theorie,
Schmelzpunkt: 188-189° C,

| | | | | | | | | |
|---|---|---|---|---|---|---|---|---|
| Ber.: | C | 71,69 | H | 7,14 | N | 2,61 | Cl | 6,61 |
| Gef.: | | 71,58 | | 7,28 | | 2,61 | | 6,67 |

Beispiel 44

2,3-Dimethoxy-7-hydroxy-7-[3-(N-methyl-N-(2-(3-benzyloxy-phenyl)-ethyl)-amino)-propen-1-yl]-6,7,8,9-tetrahydro-5H-benzocyclohepten-hydrochlorid

1,0 g (0,002 Mol) 2,3-Dimethoxy-7-hydroxy-7-[3-(N-methyl-N-(2-(3-benzyloxy-phenyl)-ethyl)-amino)-propin-1-yl]-6,7,8,9-tetrahydro-5H-benzocyclohepten werden in 15 ml Ethanol in Anwesenheit von 0,15 g Raney-Nickel 3,5 Stunden bei Raumtemperatur und 1 bar Wasserstoff hydriert. Nach Filtration und Eindampfen im Vakuum wird das Rohprodukt über 360 g Aluminiumoxid (neutral, Aktivität II-III) mit Methylenchlorid und ansteigenden Anteilen von Ethanol (bis 2 %) gereinigt. Anschließend wird in Aceton mit etherischer Salzsäure das Hydrochlorid gefällt.
Ausbeute: 180 mg (19 % der Theorie),
Schmelzpunkt: 165-166° C,

| | | | | | | | | |
|---|---|---|---|---|---|---|---|---|
| Ber.: | C | 71,44 | H | 7,31 | N | 2,60 | Cl | 6,59 |
| Gef.: | | 71,36 | | 7,49 | | 2,70 | | 6,77 |

Beispiel 45

2,3-Dimethoxy-7-hydroxy-7-[3-(N-methyl-N-(2-(3-hydroxy-phenyl)-ethyl)-amino)-propyl]-6,7,8,9-tetrahydro-5H-benzocyclohepten

Hergestellt aus 2,3-Dimethoxy-7-hydroxy-7-[3-(N-methyl-N-(2-(3-benzyloxy-phenyl)-ethyl)-amino)-propin-1-yl]-6,7,8,9-tetrahydro-5H-benzocyclohepten analog Beispiel 13.
Ausbeute: 50 % der Theorie,

| | | | | | | |
|---|---|---|---|---|---|---|
| Ber.: | C | 72,61 | H | 8,53 | N | 3,39 |
| Gef.: | | 72,44 | | 8,51 | | 3,29 |

$R_F$-Wert: 0,33 (Aluminiumoxid, Laufmittel: 5 % Ethanol in Methylenchlorid)

Beispiel 46

2,3-Dimethoxy-7-hydroxy-7-[3-(N-methyl-N-(2-(3-methansulfonyloxy-phenyl)-ethyl)-amino)-propyl]-6,7,8,9-tetrahydro-5H-benzocyclohepten-hydrochlorid

Hergestellt aus 2,3-Dimethoxy-7-hydroxy-7-[3-(N-methyl-N-(2-(3-hydroxy-phenyl)-ethyl)-amino)-propyl]-6,7,8,9-tetrahydro-5H-benzocyclohepten und Methansulfonsäurechlorid analog Beispiel 6.
-Ausbeute: 20 % der Theorie,
Schmelzpunkt: 146-148° C,

| | | | | | | | | | | |
|---|---|---|---|---|---|---|---|---|---|---|
| Ber.: | C | 59,13 | H | 7,25 | N | 2,65 | Cl | 6,71 | S | 6,07 |
| Gef.: | | 58,97 | | 7,38 | | 2,64 | | 6,63 | | 6,09 |

Beispiel 47

2,3-Dimethoxy-7-hydroxy-7-[3-(N-methyl-N-(2-phenylethyl)-amino)-propin-1-yl]-6,7,8,9-tetrahydro-5H-benzocyclohepten-hydrochlorid

Hergestellt aus 3-[N-methyl-N-(2-phenylethyl)-amino]-propin und 2,3-Dimethoxy-6,7,8,9-tetrahydro-5H-benzocyclohepten-7-on analog Beispiel 8.
Ausbeute: 97 % der Theorie,
Schmelzpunkt: 217° C,

| | | | | | | | | |
|---|---|---|---|---|---|---|---|---|
| Ber.: | C | 69,83 | H | 7,50 | N | 3,26 | Cl | 8,25 |
| Gef.: | | 69,79 | | 7,68 | | 3,17 | | 8,20 |

Beispiel 48

2,3-Dimethoxy-7-hydroxy-7-[3-(N-methyl-N-(2-phenylethyl)-amino)-propyl]-6,7,8,9-tetrahydro-5H-benzocyclohepten-hydrochlorid

Hergestellt aus 2,3-Dimethoxy-7-hydroxy-7-[3-(N-methyl-N-(2-phenylethyl)-amino)-propin-1-yl]-6,7,8,9-tetrahydro-5H-benzocyclohepten analog Beispiel 13.
Ausbeute: 47 % der Theorie,
Schmelzpunkt: 194-195° C,

| | | | | | | | | |
|---|---|---|---|---|---|---|---|---|
| Ber.: | C | 69,18 | H | 8,36 | N | 3,23 | Cl | 8,17 |
| Gef.: | | 69,00 | | 8,29 | | 3,21 | | 8,25 |

Beispiel 49

2,3-Dimethoxy-7-hydroxy-7-[3-(N-methyl-N-(3-phenylpropyl)-amino)-propin-1-yl]-6,7,8,9-tetrahydro-5H-benzocyclohepten-hydrochlorid

Hergestellt aus 3-(N-methyl-N-(3-phenylpropyl)-amino)-propin und 2,3-Dimethoxy-6,7,8,9-tetrahydro-5H-benzocyclohepten-7-on analog Beispiel 8.
Ausbeute: 76 % der Theorie,
Schmelzpunkt: 184-185° C,

| | | | | | | | | |
|---|---|---|---|---|---|---|---|---|
| Ber.: | C | 70,33 | H | 7,72 | N | 3,15 | Cl | 7,99 |
| Gef.: | | 70,26 | | 7,84 | | 3,04 | | 8,08 |

## Beispiel 50

2,3-Dimethoxy-7-hydroxy-7-[3-(N-methyl-N-(3-phenylpropyl)-amino)-propyl]-6,7,8,9-tetrahydro-5H-benzocyclohepten-hydrochlorid

Hergestellt aus 2,3-Dimethoxy-7-hydroxy-7-[3-(N-methyl-N-(3-phenylpropyl)-amino)-propin-1-yl]-6,7,8,9-tetrahydro-5H-benzocyclohepten analog Beispiel 13.
Ausbeute: 40 % der Theorie,
Schmelzpunkt: 187° C,

| | | | | | | | | |
|---|---|---|---|---|---|---|---|---|
| Ber.: | C | 69,70 | H | 8,55 | N | 3,13 | Cl | 7,91 |
| Gef.: | | 69,65 | | 8,68 | | 2,97 | | 8,06 |

## Beispiel 51

2,3-Dimethoxy-7-hydroxy-7-[3-(N-methyl-N-(2-(3-methyl-phenyl)-ethyl)-amino)-propin-1-yl]-6,7,8,9-tetrahydro-5H-benzocyclohepten-hydrochlorid

Hergestellt aus 3-(N-methyl-N-(2-(3-methyl-phenyl)-ethyl)-amino)-propin und 2,3-Dimethoxy-6,7,8,9-tetrahydro-5H-benzocyclohepten-7-on analog Beispiel 8.
Ausbeute: 57 % der Theorie,
Schmelzpunkt: 185-186° C,

| | | | | | | | | |
|---|---|---|---|---|---|---|---|---|
| Ber.: | C | 70,33 | H | 7,72 | N | 3,15 | Cl | 7,99 |
| Gef.: | | 70,18 | | 7,66 | | 3,07 | | 8,05 |

## Beispiel 52

2,3-Dimethoxy-7-hydroxy-7-[3-(N-methyl-N-(2-(3-methyl-phenyl)-ethyl)-amino)-propyl]-6,7,8,9-tetrahydro-5H-benzocyclohepten-hydrochlorid

Hergestellt aus 2,3-Dimethoxy-7-hydroxy-7-[3-(N-methyl-N-(2-(3-methyl-phenyl)-ethyl)-amino)-propin-1-yl]-6,7,8,9-tetrahydro-5H-benzocyclohepten analog Beispiel 13.
Ausbeute: 42 % der Theorie,
Schmelzpunkt: 169° C,

| | | | | | | | | |
|---|---|---|---|---|---|---|---|---|
| Ber.: | C | 69,70 | H | 8,55 | N | 3,13 | Cl | 7,91 |
| Gef.: | | 69,56 | | 8,58 | | 3,22 | | 7,94 |

Beispiel 53

2,3-Dimethoxy-7-[3-(N-methyl-N-(2-(3,4-dimethoxy-phenyl)-ethyl)-amino)-propyl]-6,7,8,9-tetrahydro-5H-benzocyclohepten-6-on

Hergestellt aus 2,3-Dimethoxy-7-[3-(N-methyl-N-(2-(3,4-dimethoxy-phenyl)-ethyl)-amino)-propin-1-yl]-6,7,8,9-tetrahydro-5H-benzocyclohepten-6-on und Wasserstoff analog Beispiel 13.
Ausbeute: 22,4 % der Theorie
IR-Spektrum (KBr) : CO 1725 $cm^{-1}$
$M^{+}$: 455

Beispiel 54

2,3-Dimethoxy-7-[3-(N-methyl-N-(2-(4-benzyloxy-phenyl)-ethyl)-amino)-propyl]-6,,7,8,9-tetrahydro-5H-benzocyclohepten-hydrochlorid

Hergestellt aus 2,3-Dimethoxy-7-[3-(N-methyl-amino)-propyl]-6,7,8,9-tetrahydro-5H-benzocyclohepten und Methansulfonsäure-2-(4-benzyloxy-phenyl)-ethylester analog Beispiel 3.
Ausbeute: 47,4 % der Theorie,
Schmelzpunkt: 160-161 °C.

Beispiel 55

7-Hydroxy-7-[3-(N-methyl-N-(2-(3,4-dimethoxy-phenyl)-ethyl)-amino)-propin-1-yl]-6,7,8,9-tetrahydro-5H-benzocyclohepten

Hergestellt aus 3-(N-Methyl-N-(2-(3,4-dimethoxy-phenyl)- ethyl)-amino)-propin und 6,7,8,9-Tetrahydro-5H-benzocyclohepten-7-on analog Beispiel 8.
Ausbeute: 56,2 % der Theorie,
Schmelzpunkt: 85-89 °C.

Beispiel 56

7-Hydroxy-7-[3-(N-methyl-N-(2-(3,4-dimethoxy-phenyl)-ethyl)-amino)-propyl]-6,7,8,9-tetrahydro-5H-benzocyclohepten-hydrochlorid

Hergestellt aus 7-Hydroxy-7-[3-(N-methyl-N-(2-(3,4-dimethoxy-phenyl)-ethyl)-amino)-propin-1-yl]-6,7,8,9-tetrahydro-5H-benzocyclohepten analog Beispiel 13.
Ausbeute: 64,6 % der Theorie,
Schmelzpunkt: 147-148 °C.

Beispiel 57

7-[3-(N-Methyl-N-(2-(3,4-dimethoxy-phenyl)-ethyl)-amino)-propyl]-6,7,8,9-tetrahydro-5H-benzocyclohepten-hydrochlorid

Hergestellt aus dem Isomerengemisch von 7-[3-(N-Methyl-N-(2-(3,4-dimethoxy-phenyl)-ethyl)-amino)-propyl]-8,9-dihydro-5H-benzocyclohepten und 7-[3-(N-Methyl-N-(2-(3,4-dimethoxy-phenyl)-ethyl)-amino)-propyliden]-5,6,8,9-tetrahydro-benzocyclohepten analog Beispiel 16.

Ausbeute: 52,8 % der Theorie,
Schmelzpunkt: 141-142° C.

Beispiel 1

Tabletten zu 10 mg 2,3-Dimethoxy-7-[3-(N-methyl-N-(2-(3,4-dimethoxy-phenyl)-ethyl)-amino)-propyl]-6,7,8,9-tetrahydro-5H-benzocyclohepten

| Zusammensetzung: | |
|---|---|
| 1 Tablette enthält: | |
| Wirksubstanz | 10,0 mg |
| Maisstärke | 57,0 mg |
| Milchzucker | 48,0 mg |
| Polyvinylpyrrolidon | 4,0 mg |
| Magnesiumstearat | 1,0 mg |
| | 120,0 mg |

Herstellungsverfahren

Der Wirkstoff, Maisstärke, Milchzucker und Polyvinylpyrrolidon werden gemischt und mit Wasser befeuchtet. Die feuchte Mischung wird durch ein Sieb mit 1,5 mm-Maschenweite gedrückt und bei ca. 45° C getrocknet. Das trockene Granulat wird durch ein Sieb mit 1,0 mm-Maschenweite geschlagen und mit Magnesiumstearat vermischt. Die fertige Mischung preßt man auf einer Tablettenpresse mit Stempeln von 7 mm Durchmesser, die mit einer Teilkerbe versehen sind, zu Tabletten. Tablettengewicht: 120 mg

Beispiel II

Dragées zu 5 mg 2,3-Dimethoxy-7-[3-(N-methyl-N-(2-(3,4-dimethoxy-phenyl)-ethyl)-amino)-propyl]-6,7,8,9-tetrahydro-5H-benzocyclohepten

| 1 Dragéekern enthält: | |
|---|---|
| Wirksubstanz | 5,0 mg |
| Maisstärke | 41,5 mg |
| Milchzucker | 30,0 mg |
| Polyvinylpyrrolidon | 3,0 mg |
| Magnesiumstearat | 0,5 mg |
| | 80,0 mg |

Herstellungsverfahren

Der Wirkstoff, Maisstärke, Milchzucker und Polyvinylpyrrolidon werden gut gemischt und mit Wasser befeuchtet. Die feuchte Masse drückt man durch ein Sieb mit 1 mm-Maschenweite, trocknet bei ca. 45° C und schlägt das Granulat anschließend durch dasselbe Sieb. Nach dem Zumischen von Magnesiumstearat werden auf einer Tablettiermaschine gewölbte Dragéekerne mit einem Durchmesser von 6 mm gepreßt. Die so hergestellten Dragéekerne werden auf bekannte Weise mit einer Schicht überzogen, die im wesentlichen aus Zucker und Talkum besteht. Die fertigen Dragées werden mit Wachs poliert.

Dragéegewicht: 130 mg

Beispiel III

Ampullen zu 5 mg 2,3-Dimethoxy-7-[3-(N-methyl-N-(2-(3,4-dimethoxy-phenyl)-ethyl)-amino)-propyl]-6,7,8,9-tetrahydro-5H-benzocyclohepten

| 1 Ampulle enthält: | |
|---|---|
| Wirksubstanz | 5,0 mg |
| Sorbit | 50,0 mg |
| Wasser für Injektionszwecke ad | 2,0 ml |

Herstellungsverfahren

In einem geeigneten Ansatzgefäß wird der Wirkstoff in Wasser für Injektionszwecke gelöst und die Lösung mit Sorbit isotonisch gestellt.
Nach Filtration über einem Membranfilter wird die Lösung unter $N_2$-Begasung in gereinigte und sterilisierte Ampullen abgefüllt und 20 Minuten im strömenden Wasserdampf autoklaviert.

Beispiel IV

Suppositorien zu 15 mg 2,3-Dimethoxy-7-[3-(N-methyl-N-(2-(3,4-dimethoxy-phenyl)-ethyl)-amino)-propyl]-6,7,8,9-tetrahydro-5H-benzocyclohepten

| 1 Zäpfchen enthält: | |
|---|---|
| Wirksubstanz | 0,015 g |
| Hartfett (z.B. Witepsol H 19 und W 45) | 1,685 g |
| | 1,700 g |

Herstellungsverfahren:

Das Hartfett wird geschmolzen. Bei 38°C wird die gemahlene Wirksubstanz in der Schmelze homogen dispergiert. Es wird auf 35°C abgekühlt und in schwach vorgekühlte Suppositorienformen ausgegossen.

Beispiel V

Tropfenlösung mit 10 mg 2,3-Dimethoxy-7-[3-(N-methyl-N-(2-(3,4-dimethoxy-phenyl)-ethyl)-amino)-propyl]-6,7,8,9-tetrahydro-5H-benzocyclohepten

| 100 ml Lösungen enthalten: | |
|---|---|
| Wirksubstanz | 0,2 g |
| Hydroxyäthylcellulose | 0,15 g |
| Weinsäure | 0,1 g |
| Sorbitlösung 70 % Trockensubstanz | 30,0 g |
| Glycerin | 10,0 g |
| Benzoesäure | 0,15 g |
| Dest.Wasser ad | 100 ml |

Herstellungsverfahren:

Dest.Wasser wird auf 70 °C erhitzt. Hierin wird unter Rühren Hydroxyäthylcellulose, Benzoesäure und Weinsäure gelöst. Es wird auf Raumtemperatur abgekühlt und hierbei das Glycerin und die Sorbitlösung unter Rühren zugegeben. Bei Raumtemperatur wird der Wirkstoff zugegeben und bis zur völligen Auflösung gerührt. Anschließend wird zur Entlüftung des Saftes unter Rühren evakuiert.

## Ansprüche

1. Benzocycloheptenderivate der Formel

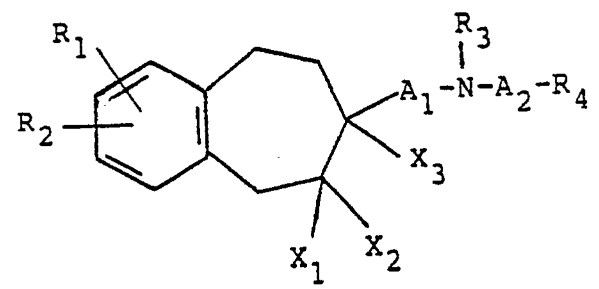

,(I)

in der
$X_1$ ein Wasserstoffatom, $X_2$ ein Wasserstoffatom und $X_3$ ein Wasserstoffatom, eine Hydroxy- oder Alkoxygruppe mit 1 bis 3 Kohlenstoffatomen oder
$X_1$ und $X_3$ zusammen eine weitere Kohlenstoff-Kohlenstoff-Bindung und $X_2$ ein Wasserstoffatom oder
$X_1$ und $X_2$ zusammen mit dem dazwischenliegenden Kohlenstoffatom eine Carbonylgruppe und $X_3$ ein Wasserstoffatom,
$A_1$ eine gegebenenfalls durch eine Alkylgruppe mit 1 bis 3 Kohlenstoffatomen substituierte geradkettige Alkylengruppe mit 3 oder 4 Kohlenstoffatomen, in welcher eine im Rest $A_1$ enthaltene mit dem Benzocycloheptenring verknüpfte Ethylengruppe durch eine Ethenylen- oder Ethinylengruppe ersetzt sein kann,
$A_2$ eine gegebenenfalls durch eine Alkylgruppe mit 1 bis 3 Kohlenstoffatomen substituierte geradkettige Alkylengruppe mit 2 bis 5 Kohlenstoffatomen, in welcher, falls n die Zahl 0 darstellt, eine in dem Rest $A_2$ enthaltene Ethylengruppe,
welche mit dem Rest $R_4$ verknüpft ist, durch eine Ethenylengruppe ersetzt sein kann,
$R_1$ ein Wasserstoff- oder Halogenatom, eine Trifluormethyl-, Nitro-, Amino-, Alkylamino-, Dialkylamino-, Alkyl-, Hydroxy-, Alkoxy- oder Phenylalkoxygruppe,
$R_2$ ein Wasserstoffatom oder Halogenatom, eine Hydroxy-, Alkoxy-, Phenylalkoxy- oder Alkylgruppe, oder
$R_1$ und $R_2$ zusammen eine Alkylendioxygruppe mit 1 oder 2 Kohlenstoffatomen,
$R_3$ ein Wasserstoffatom, eine Alkylgruppe oder eine Alkenylgruppe mit 3 bis 5 Kohlenstoffatomen und
$R_4$ eine Gruppe der Formel

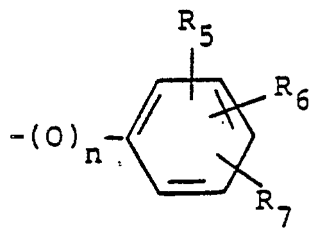

, wobei

n die Zahl 0 oder 1,
$R_5$ ein Wasserstoff- oder Halogenatom, eine Alkyl-, Nitro-, Amino-, Alkylamino-, Dialkylamino-, Alkanoylamino-, Alkoxycarbonylamino-, Alkylsulfonylamino, Bis(alkylsulfonyl)-amino-, N-Alkyl-alkylsulfonylamino, Cyano-, Alkylmercapto-, Alkylsulfinyl- oder Alkylsulfonylgruppe oder eine gegebenenfalls durch eine Alkyl-, Phenylalkyl-, 2-Hydroxyäthyl-, 3-Hydroxy-n-propyl, 2-Hydroxy-n-propyl-, Alkylsulfonyl, Cyanoalkyl-, Alkoxycarbonyl-, Hydroxycarbonylalkyl-, Alkoxycarbonylalkyl-, Trifluormethyl-, Difluormethyl oder Trifluormethylsulfonylgruppe substituierte Hydroxygruppe,
$R_6$ ein Wasserstoff- oder Halogenatom, eine Alkyl-, Hydroxy-, Alkoxy-, Cyano- oder Trifluormethylgruppe oder
$R_5$ und $R_6$ zusammen eine Alkylendioxygruppe mit 1 oder 2 Kohlenstoffatomen und
$R_7$ ein Wasserstoff- oder Halogenatom, eine Alkyl- oder Alkoxygruppe bedeuten, wobei alle vorstehend erwähnten Alkyl- oder Alkoxygruppen jeweils 1 bis 3 Kohlenstoffatome und die vorstehend erwähnten Alkanoylgruppen jeweils 2 oder 3 Kohlenstoffatome enthalten können, deren Enantiomeren, deren Diastereomeren und deren Säureadditionssalze, insbesondere deren physiologisch verträgliche Säureadditionssalze mit anorganischen oder organischen Säuren.

2. Benzocycloheptenderivate der Formel 1 gemäß Anspruch 1, in der
$X_1$ ein Wasserstoffatom, $X_2$ ein Wasserstoffatom und $X_3$ ein Wasserstoffatom, eine Hydroxy- oder Methoxygruppe oder
$X_1$ und $X_3$ zusammen eine weitere Kohlenstoff-Kohlenstoff-Bindung und $X_2$ ein Wasserstoffatom oder
$X_1$ und $X_2$ zusammen mit dem dazwischenliegenden Kohlenstoffatom eine Carbonylgruppe und $X_3$ ein Wasserstoffatom,
$A_1$ eine n-Propylengruppe, in welcher die mit dem Cycloheptenring verknüpfte Ethylengruppe durch eine Ethenylen- oder Ethinylengruppe ersetzt sein kann,
$A_2$ eine Ethylen- oder n-Propylengruppe oder eine n-Propylengruppe, in der, falls n die Zahl 0 darstellt, eine im Rest $A_2$ enthaltene Ethylengruppe, welche mit dem Rest $R_4$ verknüpft ist, durch eine Ethenylengruppe ersetzt ist,
$R_1$ ein Wasserstoffatom, eine Methyl- oder Methoxygruppe,
$R_2$ ein Wasserstoffatom, eine Methyl- oder eine Methoxygruppe,
$R_3$ die Methylgruppe,
n die Zahl 0 oder 1,
$R_5$ ein Wasserstoff-, Fluor-, Chlor- oder Bromatom, eine Hydroxy-, Methoxy-, Cyano-, Methyl-, Nitro-, Amino-, Methylsulfonyloxy-, Trifluormethylsulfonyloxy- oder Benzyloxygruppe,
$R_6$ ein Wasserstoff-, Chlor oder Bromatom oder eine Methoxygruppe und
$R_7$ ein Wasserstoff-, Chlor- oder Bromatom bedeuten, deren Enantiomere, deren Diastereomere und deren Säureadditionssalze.

3. Benzocycloheptenderivate der Formel I gemäß Anspruch 1, in der
$X_1$ ein Wasserstoffatom, $X_2$ ein Wasserstoffatom und $X_3$ ein Wasserstoffatom oder eine Hydroxygruppe oder
$X_1$ und $X_3$ zusammen eine weitere Kohlenstoff-Kohlenstoff-Bindung und $X_2$ ein Wasserstoffatom oder
$X_1$ und $X_2$ zusammen mit dem dazwischenliegenden Kohlenstoffatom eine Carbonylgruppe und $X_3$ ein Wasserstoffatom,
$R_1$ eine Methoxygruppe,
$R_2$ eine Methoxygruppe,
$R_3$ eine Methylgruppe,
$A_1$ eine n-Propylengruppe,
$A_2$ eine Ethylen- oder n-Propylengruppe,
$R_5$ eine Methoxy- oder Methylsulfonyloxygruppe,

$R_6$ ein Wasserstoffatom oder eine Methoxygruppe,
$R_7$ ein Wasserstoffatom und
n die Zahl 0 oder 1 bedeuten, deren Enantiomere, deren Diastereomere und deren Säureadditionssalze.

4. Als Benzocycloheptenderivate der Formel I gemäß Anspruch 1:
2,3-Dimethoxy-7-[3-(N-methyl-N-(2-(3,4-dimethoxy-phenyl)-ethyl)-amino)-propyl]-6,7,8,9-tetrahydro-5H-benzocyclohepten,
2,3-Dimethoxy-7-[3-(N-methyl-N-(2-(4-methoxy-phenyl)-ethyl)-amino)-propyl]-6,7,8,9-tetrahydro-5H-benzocyclohepten,
2,3-Dimethoxy-7- [3-(N-methyl-N-(2-(3-methoxy-phenyl)-ethyl)-amino)-propyl]-6,7,8,9-tetrahydro-5H-benzocyclohepten,
2,3-Dimethoxy-7-hydroxy-7-[3-(N-methyl-N-(2-(3-methoxy-phenyl)-ethyl)-amino)-propyl]-6,7,8,9-tetrahydro-5H-benzocyclohepten,
2,3-Dimethoxy-7-hydroxy-7-[3-(N-methyl-N-(3-(3-methoxy-phenoxy)-propyl)-amino)-propyl]-6,7,8,9-tetrahydro-5H-benzocyclohepten und
2,3-Dimethoxy-7-hydroxy-7-[3-(N-methyl-N-(2-(3-methansulfonyloxy-phenyl)-ethyl)-amino)-propyl]-6,7,8,9-tetrahydro-5H-benzocyclohepten,
sowie deren Enantiomere, deren Diastereomere und deren Säureadditionssalze.

5. 2,3-Dimethoxy-7-[3(N-methyl-N-(2-(3,4-dimethoxy-phenyl)-ethyl)-amino)-propyl]-6,7,8,9-tetrahydro-5H-benzocyclohepten, dessen Enantiomere, dessen Diastereomere und dessen Säureadditionssalze.

6. Physiologisch verträgliche Säureadditionssalze der Verbindungen nach mindestens einem der Ansprüche 1 bis 5 mit anorganischen oder organischen Säuren.

7. Arzneimittel, enthaltend eine Verbindung der Formel I nach mindestens einem der Ansprüche 1 bis 5 oder dessen physiologisch verträgliches Säureadditionssalz gemäß Anspruch 6 neben einem oder mehreren inerten Trägerstoffen und/oder Verdünnungsmitteln.

8. Arzneimittel gemäß Anspruch 7 geeignet zur Behandlung und Prophylaxe ischämischer Herzerkrankungen und zur Behandlung von Sinustachycardien.

9. Verfahren zur Herstellung eines Arzneimittels gemäß Anspruch 7 oder 8, dadurch gekennzeichnet, daß auf nichtchemischem Wege eine Verbindung nach mindestens einem der Ansprüche 1 bis 5 oder dessen physiologisch verträgliches Säureadditionssalz gemäß Anspruch 6 in einen oder mehrere inerten Trägerstoffe und/oder Verdünnungsmittel eingearbeitet wird.

10. Verfahren zur Herstellung der Benzocycloheptenderivate nach mindestens einem der Ansprüche 1 bis 6, dadurch gekennzeichnet, daß
a) eine Verbindung der Formel

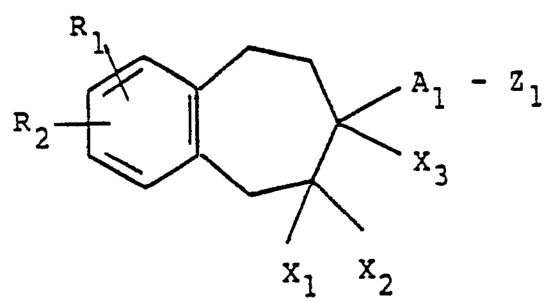

,(II)

mit einer Verbindung der Formel
$Z_2 - A_2 - R_4$ (III)
in denen
$R_1$, $R_2$, $R_4$, $A_1$, $A_2$ und $X_1$ bis $X_3$ wie mindestens in einem der Ansprüche 1 bis 5 definiert sind,
einer der Reste $Z_1$ oder $Z_2$ eine $R_3$-NH-Gruppe, wobei $R_3$ wie mindestens in einem der Ansprüche 1 bis 5 definiert ist, und
der andere der Reste $Z_1$ oder $Z_2$ eine nukleophile Austrittsgruppe darstellt, umgesetzt wird oder
b) zur Herstellung von Verbindungen der Formel I, in der $X_3$ ein Wasserstoffatom darstellt, eine Verbindung der Formel

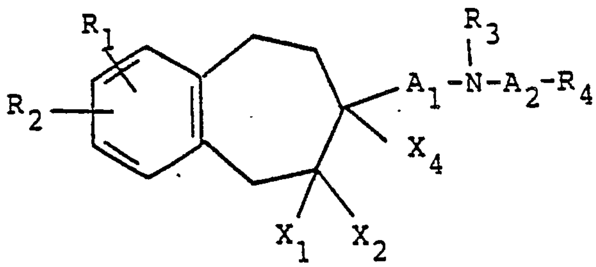

,(IV)

in der
$R_1$ bis $R_4$, $X_1$, $X_2$, $A_1$ und $A_2$ wie mindestens in einem der Ansprüche 1 bis 5 definiert sind,
wobei jedoch
$X_4$ eine Hydroxygruppe oder
$X_1$ und $X_4$ zusammen eine weitere Kohlenstoff-Kohlenstoff-Bindung oder
$X_4$ zusammen mit einem Wasserstoffatom des benachbarten gesättigten Kohlenstoffatoms des Restes $A_1$ eine Kohlenstoff-Kohlenstoff-Bindung darstellen muß, katalytisch hydriert wird oder
c) zur Herstellung von Verbindungen der Formel I, in der $X_1$ und eine weitere Kohlenstoff-Kohlenstoff-Bindung darstellen, ein Rest $HZ_3$ von einer Verbindung der Formel

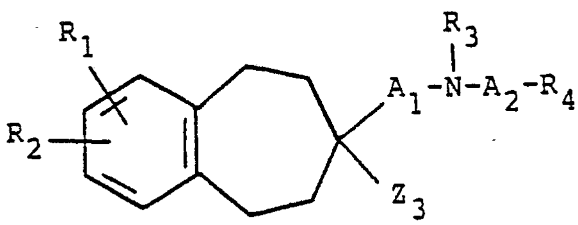

,(V)

in der
$R_1$ bis $R_4$, $A_1$ und $A_2$ wie mindestens in einem der Ansprüche 1 bis 5 definiert sind und
$Z_3$ eine abspaltbare Gruppe darstellt, abgespalten wird oder
d) zur Herstellung von Verbindungen der Formel I, in der $X_3$ die Hydroxygruppe darstellt, eine Verbindung der Formel

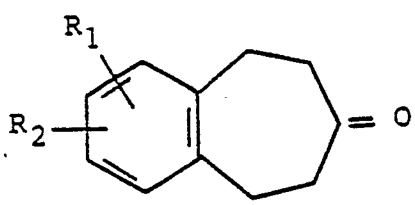

,(VI)

in der
$R_1$ und $R_2$ wie mindestens in einem der Ansprüche 1 bis 5 definiert sind, mit einer Verbindung der Formel

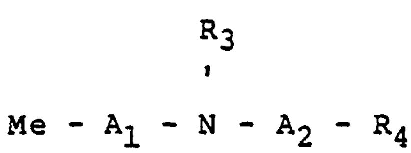

,(VII)

in der
$R_3$, $R_4$, $A_1$ und $A_2$ wie mindestens in einem der Ansprüche 1 bis 5 definiert sind und
Me ein Alkaliatom oder eine MgHal-Gruppe darstellt, wobei Hal ein Chlor-, Brom- oder Jodatom bedeutet, umgesetzt wird oder

e) zur Herstellung von Verbindungen der Formel I, in der $R_5$ eine Alkylamino-, Dialkylamino-, Alkanoylamino-, Alkoxycarbonylamino-, Alkylsulfonylamino-, Bis(alkylsulfonyl-amino-, N-Alkyl-alkylsulfonylamino-, Alkylmercapto-, Alkoxy-, Alkoxycarbonyloxy-, Hydroxycarbonylalkoxy-, Alkoxycarbonylalkoxy-, Phenylalkoxy-, Trifluormethoxy-, Difluormethoxy-, Cyanoalkoxy-, Alkylsulfonyloxy- oder Trifluormethylsulfonyloxygruppe darstellt, eine Verbindung der Formel

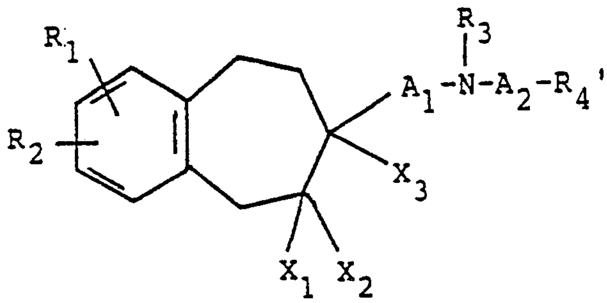

,(VIII)

in der

$R_1$ bis $R_3$, $A_1$, $A_2$ und $X_1$ bis $X_3$ wie mindestens in einem der Ansprüche 1 bis 5 definiert sind und

$R_4'$ eine Gruppe der Formel

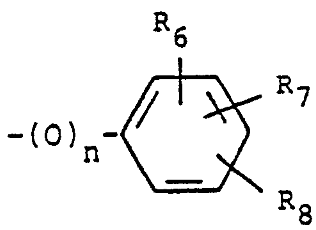

darstellt, wobei

$R_6$, $R_7$ und n wie mindestens in einem der Ansprüche 1 bis 5 definiert sind und

$R_8$ eine Hydroxy-, Amino- oder Alkylaminogruppe mit 1 bis 3 Kohlenstoffatomen darstellt, mit einer Verbindung der Formel

$Z_4 - R_9$ ,(IX)

in der

$Z_4$ eine nukleophile Austrittsgruppe und

$R_9$ eine Alkyl-, Alkanoyl-, Alkoxycarbonyl, Hydroxycarbonylalkyl-, Alkoxycarbonylalkyl, Alkylsulfonyl, Phenylalkyl-, Trifluormethyl-, Difluormethyl oder Cyanoalkylgruppe bedeuten, wobei die vorstehend erwähnten Alkyl- und Alkoxyteile jeweils 1 bis 3 Kohlenstoffatome bzw. der Alkanoylteil 2 oder 3 Kohlenstoffatome enthalten kann, umgesetzt wird oder

f) eine Verbindung der Formel

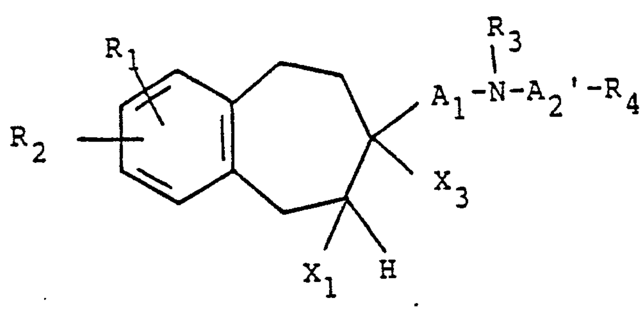

,(X

in der

$R_1$ bis $R_4$, $A_1$, $X_1$ und wie mindestens in einem der Ansprüche 1 bis 5 definiert sind und

$A_2'$ eine gegebenenfalls durch eine Alkylgruppe mit 1 bis 3 Kohlenstoffatomen substituierte geradkettige

Alkylengruppe mit 2 bis 4 Kohlenstoffatomen darstellt, in welcher eine mit dem Stickstoffatom der $R_3$-N< Gruppe verknüpfte Methylengruppe durch eine Carbonylgruppe ersetzt ist, reduziert wird oder
g) zur Herstellung von Verbindungen der Formel I, in der $A_1$ eine gegebenenfalls durch eine Alkylgruppe mit 1 bis 3 Kohlenstoffatomen substituierte geradkettige Alkylengruppe mit 3 oder 4 Kohlenstoffatomen darstellt, in welcher eine mit dem Benzocycloheptenring verknüpfte Ethylengruppe durch eine Ethenylengruppe ersetzt sein kann, eine Verbindung der Formel

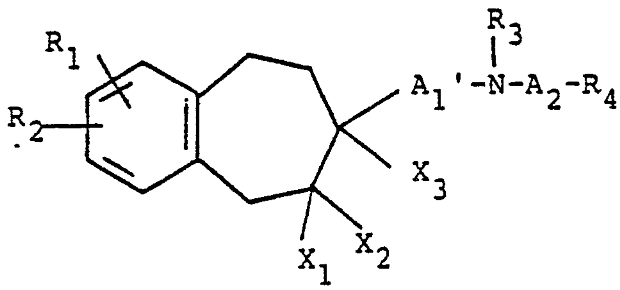

, (XI)

in der
$R_1$ bis $R_4$, $A_2$ und $X_1$ bis $X_3$ wie mindestens in einem der Ansprüche 1 bis 5 definiert sind und
$A_1'$ eine gegebenenfalls durch eine Alkylgruppe mit 1 bis 3 Kohlenstoffatomen substituierte geradkettige Alkylengruppe mit 3 oder 4 Kohlenstoffatomen darstellt, in welcher eine im Rest $A_1'$ enthaltene mit dem Benzocycloheptenring verknüpfte Ethylengruppe durch eine Ethenylen- oder Ethinylengruppe ersetzt ist, katalytisch hydriert wird,
wobei erforderlichenfalls eine bei den Umsetzungen a) bis g) zum Schutze von reaktiven Gruppen verwendete Schutzgruppe anschließend abgespalten wird
und gewünschtenfalls anschließend eine so erhaltene Verbindung der Formel I, in der $R_5$ eine Benzyloxygruppe darstellt, mittels Entbenzylierung in die entsprechende Hydroxyverbindung übergeführt wird und/oder
eine so erhaltene Verbindung der Formel I, welche mindestens ein chirales Zentrum enthält, in ihre Diastereomeren oder in ihre Enantiomeren aufgetrennt wird und/oder
eine so erhaltene Verbindung der Formel I in ihre Säureadditionssalze, insbesondere in ihre physiologisch verträglichen Säureadditionssalze mit anorganischen oder organischen Säuren übergeführt wird.